# EUROPEAN PATENT APPLICATION

(11) **EP 1 634 605 A2**
(43) Date of publication of application: **15.03.2006**
(21) Application number: 05110709.2
(22) Date of filing: 08.03.2001
(51) Int. Cl.: A61K 38/26, C07K 14/605, A61P 3/06

(54) **Treatment of dyslipidemia in a patient having type 2 diabetes**

(30) Priority: 08.03.2000 DK 200000375
(62) Divisional of application: 01909571.0
(71) Applicant: NOVO NORDISK A/S, 2880 Bagsvaerd (DK)
(72) Inventor: Knudsen, Liselotte Bjerre, 4400 Kalundborg (DK)
(74) Representative: Jensen, Dorte Kold

(57) **Abstract**

The present invention relates to a method for lowering serum lipids, eg. triglycerides and/or cholesterol in a subject comprising administering a GLP-1 agonist to said subject.

## Description

The present invention relates to a method for lowering serum lipids, eg. triglycerides and/or cholesterol in a subject comprising administering a GLP-1 agonist to said subject.

### Background

Lipids (e.g. cholesterol, cholesterol-ester and triglycerides) are transported in plasma in so called lipoproteins. These lipoproteins consist of a spherical hydrophobic core of triglycerides and cholesterol esters surrounded by an amphipathic monolayer of phospholipids, cholesterol and apolipoproteins. Lipoproteins are classified according to size/density and nature of associated lipoproteins. The principal classes of lipoproteins are the following: Chylomicrons are large gut-synthesized particles that transport intestinal absorbed lipids to the liver and adipose tissue. The liver secretes large triglyceride-rich particles known as VLDL (very-low-density lipoproteins. These particles are modified peripherally, initially to IDL (intermediate-density lipoprotein) and later to LDL (low-density-lipoprotein). The latter is cholesterol-rich and it may finally be transformed to small, dense LDL. The liver also secretes cholesterol-rich HDL (high-density-lipoprotein) particles. Vast amount of data links abnormalities in plasma lipoprotein to development of atherosclerosis. Clinical manifestations of atherosclerosis are the ischemic heart diseases (IHD) like stable and unstable angina pectoris, myocardial infarction and cardiac insufficiency. Other manifestations are cerebrovascular diseases like stroke and cerebral hemorrhage. Still other manifestations are peripheral artery diseases like intermittent claudication, and aneurisms of aorta and other large arteries.

The discussion on association between lipoproteins and development of atherosclerosis has for many years focus on the importance of LDL cholesterol. Elevated LDL cholesterol is now thought to be a causal factor in the development of atherosclerosis and its associated diseases It has been increasingly accepted, however that increased LDL cholesterol may not be the only lipid and lipoprotein that constitutes a risk factor for the development of atherosclerosis. Epidemiological studies have highlighted additional risk factors among the lipoproteins. Elevated triglycerides (eg. elevated VLDL) (Hokanson,JE J,Cardiovasc.Risk, 1996; 3:213-219) and low concentration of HDL cholesterol (Uusitupa, MIJ Circulation 1990; 82: 27-36) have been identified as important risk factors.

Furthermore, changes in particle size of lipoproteins - not reflected in quantitative measurements of said particles - may constitute risk factors for atherosclerosis. Increased concentration of "small, dense LDL" particles, even in situations with normal LDL cholesterol, may be a very significant risk factor (Griffin, BA Atherosclerosis 1994; 106: 241-353). Clustering of risk factors may be seen in certain situations, eg. diabetic patients very often have dyslipidemia characterized by elevated triglycerides, low HDL cholesterol, normal LDL cholesterol but increased amounts of small dense, LDL particles. In other situation, lipoprotein abnormalities may occur as isolated events, eg. elevated LDL cholesterol or decreased HDL cholesterol.

Still another lipoprotein that constitutes a risk factor for atherosclerosis is lipoprotein a (Lp(a)). Lipoprotein(a) [Lp(a)] represents an LDL-like particle to which the Lp(a)-specific apol-ipoprotein(a) is linked via a disulfide bridge. It has gained considerable interest as a genetically determined risk factor for atherosclerotic vascular disease. Several studies have described a correlation between elevated Lp(a) plasma levels and coronary heart disease, stroke, and peripheral atherosclerosis (Kronenberg, F Crit.Rev.Clin.Lab.Sci. 1996; 6: 495-543).

If one concurs with the notion that atherosclerosis and associated cardiovascular diseases are related to abnormal levels of plasma lipids and lipoproteins, then lowering them represent a desirable therapeutic goal. Lowering of LDL cholesterol through treatment with statins improves on the mortality from cardiovascular diseases (Lancet 1994; 344: 1383-1389). Lowering of triglycerides through treatment with fibrates may also lower the incidence of cardiovascular diseases (Frick, MA New Engl.J.Med. 1987; 317:1237-1245).

Human GLP-1 is a 37 amino acid residue peptide originating from preproglucagon which is synthesised *i.a.* in the L-cells in the distal ileum, in the pancreas and in the brain (see i.a. Ørskov C. Glucagon-like peptide-1, a new hormone of the enteroinsular axis. Diabetolo-gia 1992; 35:701-711.). Processing of preproglucagon to give GLP-1 (7-36)amide, GLP-1 (7-37) and GLP-2 occurs mainly in the L-cells. A simple system is used to describe fragments and analogues of this peptide. Thus, for example, Gly⁸-GLP-1(7-37) designates a fragment of GLP-1 formally derived from GLP-1 by deleting the amino acid residues Nos. 1 to 6 and substituting the naturally occurring amino acid residue in position 8 (Ala) by Gly. Similarly, Lys³⁴(N^{ε-}tetradecanoyl)-GLP-1 (7-37) designates GLP-1 (7-37) wherein the ε-amino group of the Lys residue in position 34 has been tetradecanoylated.

One object of the present invention is to provide compositions which can effectively be used in the treatment or prophylaxis of dyslipidaemia, hyperlipoproteinaemia, hypertriglyceridaemia, hyperlipidaemia or hypercholesterolaemia.

Another object of the present invention is to provide compositions which can effectively be used in the treatment or prophylaxis of arteriosclerosis including atherosclerosis.

Other objects of the present invention will become apparent upon reading the present description.

### Description of the invention

It has been discovered that GLP-1 lowers plasma levels of lipids, such as triglycerides, cholesterol, and non-estified fatty acid (NEFA) lipids, on a long-term basis.

Accordingly, the present invention relates to a method of lowering total serum lipids, which method comprises administering to a subject an effective amount of a GLP-1 agonist.

The invention also relates to a use of a GLP-1 agonist for the manufacture of a medicament for lowering total serum lipids.

In a further aspect the present invention relates to a method for treating a human having a disease-state which is alleviated by lowering total serum lipids, comprising administering to said human an effective amount of a GLP-1 agonist.

In a further aspect the invention relates to a use of a GLP-1 agonist for the manufacture of a medicament for treating a human having a disease-state which is alleviated by lowering total serum lipids.

In a further aspect the invention relates to a method of lowering LDL, which method comprises administering to a subject an effective amount of a GLP-1 agonist.

In a further aspect the invention relates to a use of a GLP-1 agonist for the manufacture of a medicament for lowering LDL.

In a further aspect the present invention relates to a method for treating a human having a disease-state which is alleviated by lowering LDL, comprising administering to said human an effective amount of a GLP-1 agonist.

In a further aspect the invention relates to a use of a GLP-1 agonist for the manufacture of a medicament for treating a human having a disease-state which is alleviated by lowering LDL.

In a further aspect the present invention relates to a method of lowering small, dense LDL, which method comprises administering to a subject an effective amount of a GLP-1 agonist.

In a further aspect the invention relates to a use of a GLP-1 agonist for the manufacture of a medicament for lowering smal, dense LDL.

In a further aspect the present invention relates to a method for treating a human having a disease-state which is alleviated by lowering smal, dense LDL, comprising administering to said human an effective amount of a GLP-1 agonist.

In a further aspect the invention relates to a use of a GLP-1 agonist for the manufacture of a medicament for treating a human having a disease-state which is alleviated by lowering smal, dense LDL.

In a further aspect the invention relates to a method of lowering VLDL, which method comprises administering to a subject an effective amount of a GLP-1 agonist.

In a further aspect the invention relates to a use of a GLP-1 agonist for the manufacture of a medicament for lowering VLDL.

In a further aspect the present invention relates to a method for treating a human having a disease-state which is alleviated by lowering VLDL, comprising administering to said human an effective amount of a GLP-1 agonist.

In a further aspect the invention relates to a use of a GLP-1 agonist for the manufacture of a medicament for treating a human having a disease-state which is alleviated by lowering VLDL.

In a further aspect the invention relates to a method of lowering triglycerides, which method comprises administering to a subject an effective amount of a GLP-1 agonist.

In a further aspect the invention relates to a use of a GLP-1 agonist for the manufacture of a medicament for lowering triglycerides.

In a further aspect the present invention relates to a method for treating a human having a disease-state which is alleviated by lowering triglycerides, comprising administering to said human an effective amount of a GLP-1 agonist.

In a further aspect the invention relates to a use of a GLP-1 agonist for the manufacture of a medicament for treating a human having a disease-state which is alleviated by lowering triglycerides.

In a further aspect the invention relates to a method of lowering cholesterol, which method comprises administering to a subject an effective amount of a GLP-1 agonist.

In a further aspect the invention relates to a use of a GLP-1 agonist for the manufacture of a medicament for lowering cholesterol.

In a further aspect the present invention relates to a method for treating a human having a disease-state which is alleviated by lowering cholesterol, comprising administering to said human an effective amount of a GLP-1 agonist.

In a further aspect the invention relates to a use of a GLP-1 agonist for the manufacture of a medicament for treating a human having a disease-state which is alleviated by lowering cholesterol.

In a further aspect the invention relates to a method of increasing HDL, which method comprises administering to a subject an effective amount of a GLP-1 agonist.

In a further aspect the invention relates to a use of a GLP-1 agonist for the manufacture of a medicament for increasing HDL.

In a further aspect the present invention relates to a method for treating a human having a disease-state which is alleviated by increasing HDL, comprising administering to said human an effective amount of a GLP-1 agonist.

In a further aspect the invention relates to a use of a GLP-1 agonist for the manufacture of a medicament for treating a human having a disease-state which is alleviated by increasing HDL.

In a further aspect the invention relates to a method of lowering the LDL/HDL ratio, which method comprises administering to a subject an effective amount of a GLP-1 agonist.

In a further aspect the invention relates to a use of a GLP-1 agonist for the manufacture of a medicament for lowering the LDL/HDL ratio.

In a further aspect the invention relates to a method of lowering fatty acids, which method comprises administering to a subject an effective amount of a GLP-1 agonist.

In a further aspect the invention relates to a use of a GLP-1 agonist for the manufacture of a medicament for lowering fatty acids.

In a further aspect the invention relates to a method of lowering free fatty acids (FFA), which method comprises administering to a subject an effective amount of a GLP-1 agonist.

In a further aspect the invention relates to a use of a GLP-1 agonist for the manufacture of a medicament for lowering FFA.

In a further aspect the invention relates to a method of lowering non-estified fatty acid (NEFA), which method comprises administering to a subject an effective amount of a GLP-1 agonist.

In a further aspect the invention relates to a use of a GLP-1 agonist for the manufacture of a medicament for lowering NEFA.

In a further aspect the present invention relates to use of a GLP-1 agonist for the manufacture of a medicament for lowering plasma levels of Lp(a) in a human.

In a further aspect the present invention also relates to use of a GLP-1 agonist for the manufacture of a medicament for inhibiting generation of apo(a) in a human.

In a further aspect the present invention relates to use of a GLP-1 agonist for the manufacture of a medicament for treating a disease-state which is alleviated by reducing plasma levels of Lp(a) in a human or inhibiting generation of apo(a) in a human.

In a further aspect the present invention relates to use of a GLP-1 agonist for the manufacture of a medicament for treating premature occlusive arterial disease in a human which is alleviated by reducing plasma levels of Lp(a) in a human or inhibiting generation of apo(a) in a human.

In another aspect the present invention is directed to a method of inhibiting generation of apo(a) in vitro or in vivo by administering a GLP-1 agonist.

Moreover, the present invention relates to a method of lowering plasma levels of Lp(a) in a human, comprising administering to said human an effective amount of a GLP-1 agonist.

Furthermore, the present invention relates to a method of inhibiting generation of apo(a) in a human, comprising administering to said human an effective amount of a GLP-1 agonist.

Furthermore, the present invention relates to a method for treating a human having a disease-state which is alleviated by lowering plasma levels of Lp(a) or inhibiting generation of apo(a), comprising administering to said human an effective amount of a GLP-1 agonist.

Furthermore, the present invention relates to a method for treating premature occlusive arterial disease in a human which is alleviated by lowering plasma levels of Lp(a) or inhibiting generation of apo(a), comprising administering to said human an effective amount of a GLP-1 agonist.

In a further aspect the invention relates to a method of treating a disease selected from cerebrovascular diseases, stroke, cerebral hemorrhage, coronary heart disease, coronary artery disease, diabetic vasculopathy, atherosclerosis, peripheral atherosclerosis, arteriosclerosis, ischemic heart disease, stable and unstable angina pectoris, cardiac insufficiency, myocardial infarction, restenosis, peripheral artery disease, intermittent claudication, aneurisms of aorta and other large arteries, or bypass graft stenosis, which method comprises administering to a subject an effective amount of a GLP-1 agonist. Each of these diseases is considered an individual embodiment of the invention.

In a further aspect the invention relates to a use of a GLP-1 agonist for the manufacture of a medicament for treating a disease selected from cerebrovascular diseases, stroke, cerebral hemorrhage, coronary heart disease, coronary artery disease, diabetic vasculopathy, atherosclerosis, peripheral atherosclerosis, arteriosclerosis, ischemic heart disease, stable and unstable angina pectoris, cardiac insufficiency, myocardial infarction, restenosis, peripheral artery disease, intermittent claudication, aneurisms of aorta and other large arteries, or bypass graft stenosis. Each of these diseases is considered an individual embodiment of the invention.

In a further aspect the invention relates to a method of treating cardiovascular diseases which method comprises administering to a subject an effective amount of a GLP-1 agonist.

In a further aspect the invention relates to a use of a GLP-1 agonist for the manufacture of a medicament for treating cardiovascular diseases.

In a further aspect the invention relates to a method of treating a disease selected from hyperlipoproteinaemia, hypertriglyceridaemia, hyperlipidaemia, or hypercholesterolaemia, which method comprises administering to a subject an effective amount of a GLP-1 agonist. Each of these diseases is considered an individual embodiment of the invention.

In a further aspect the invention relates to a use of a GLP-1 agonist for the manufacture of a medicament for treating a disease selected from hyperlipidaemia, hyperlipoproteinaemia, hypertriglyceridaemia, or hypercholesterolaemia. Each of these diseases is considered an individual embodiment of the invention.

In a further aspect the invention relates to a method of treating dyslipideamia which method comprises administering to a subject an effective amount of a GLP-1 agonist.

In a further aspect the invention relates to a use of a GLP-1 agonist for the manufacture of a medicament for treating dyslipidemia.

In a further aspect the invention relates to a use of a GLP-1 agonist for the manufacture of a medicament for treating dyslipidemia in a diabetic patient.

In a further aspect the invention relates to a use of a GLP-1 agonist for the manufacture of a medicament for treating dyslipidemia in a patient having type 1 diabetes, also known as IDDM.

In a further aspect the invention relates to a use of a GLP-1 agonist for the manufacture of a medicament for treating dyslipidemia in a patient having type 2 diabetes, also known as NIDDM.

In a further aspect the invention relates to a method of treating arteriosclerosis which method comprises administering to a subject an effective amount of a GLP-1 agonist.

In a further aspect the invention relates to a use of a GLP-1 agonist for the manufacture of a medicament for treating arteriosclerosis.

In a further aspect the invention relates to a method of treating atherosclerosis which method comprises administering to a subject an effective amount of a GLP-1 agonist.

In a further aspect the invention relates to a use of a GLP-1 agonist for the manufacture of a medicament for treating atherosclerosis.

In a further aspect the invention relates to a method for the treatment of patients in a need of an anticoagulative treatment, e.g. following a coronary thrombosis or after surgery, which method comprises administering to a subject an effective amount of a GLP-1 agonist.

In a further aspect the invention relates to a use of a GLP-1 agonist for the manufacture of a medicament for anticoagulative treatment, e.g. following a coronary thrombosis or after surgery.

The subject or patient is preferably a mammal, more preferably a human.

The use according any one of the above uses in a regimen which additionally comprises treatment with human growth hormone, a growth hormone releasing agent or a growth factor such as prolactin or placental lactogen; the use of human growth hormone, a growth hormone releasing agent or a growth factor such as prolactin or placental lactogen for the manufacture of a medicament for lowering serum lipids in a subject; the use of human growth hormone, a growth hormone releasing agent or a growth factor such as prolactin or placental lactogen for the manufacture of a medicament for lowering serum lipids in a subject.

In a further aspect the invention relates to a use of a GLP-1 agonist, preferably Arg³⁴, Lys²⁶(N^{ε}-(γ-Glu(N^{α}-hexadecanoyl)))-GLP-1(7-37), for the manufacture of a medicament for treating diabetes type 1 in a regimen which additionally comprises treatment with insulin.

In a further aspect the invention relates to a use of a GLP-1 agonist, preferably Arg³⁴, Lys²⁶(N^{ε}-(γ-Glu(N^{α}-hexadecanoyl)))-GLP-1(7-37), for the manufacture of a medicament for treating diabetes type 2 in a regimen which additionally comprises treatment with insulin.

In a further aspect the invention relates to a use of a GLP-1 agonist, preferably Arg³⁴, Lys²⁶(N^{ε}-(γ-Glu(N^{α}-hexadecanoyl)))-GLP-1(7-37), for the manufacture of a medicament for treating dyslipidemia in a patient having type 2 diabetes, in a regimen which additionally comprises treatment with insulin.

In a further aspect the invention relates to a use of a GLP-1 agonist, preferably Arg³⁴, Lys²⁶(N^{ε}-(γ-Glu(N^{α}-hexadecanoyl)))-GLP-1(7-37), for the manufacture of a medicament for treating diabetes type 2 in a regimen which additionally comprises treatment with a sulphonylurea, such as any one of tolbutamide, glibenclamide, glipizide or glicazide.

In a further aspect the invention relates to a use of a GLP-1 agonist, preferably Arg³⁴, Lys²⁶(N^{ε}-(γ-Glu(N^{α}-hexadecanoyl)))-GLP-1(7-37), for the manufacture of a medicament for treating dyslipidemia in a patient having type 2 diabetes, in a regimen which additionally comprises treatment with a sulphonylurea, such as any one of tolbutamide, glibenclamide, glipizide or glicazide.

In a further aspect the invention relates to a use of a GLP-1 agonist, preferably Arg³⁴, Lys²⁶(N^{ε}-(γ-Glu(N^{α}-hexadecanoyl)))-GLP-1(7-37), for the manufacture of a medicament for treating diabetes type 2 in a regimen which additionally comprises treatment with a biguanide such as metformin.

In a further aspect the invention relates to a use of a GLP-1 agonist, preferably Arg³⁴, Lys²⁶(N^{ε}-(γ-Glu(N^{α}-hexadecanoyl)))-GLP-1(7-37), for the manufacture of a medicament for treating dyslipidemia in a patient having type 2 diabetes, in a regimen which additionally comprises treatment with a biguanide such as metformin.

In a further aspect the invention relates to a use of a GLP-1 agonist, preferably Arg³⁴, Lys²⁶(N^{ε}-(γ-Glu(N^{α}-hexadecanoyl)))-GLP-1(7-37), for the manufacture of a medicament for treating diabetes type 2 in a regimen which additionally comprises treatment with such as repaglinide.

In a further aspect the invention relates to a use of a GLP-1 agonist, preferably Arg³⁴, Lys²⁶(N^{ε}-(γ-Glu(N^{α}-hexadecanoyl)))-GLP-1(7-37), for the manufacture of a medicament for treating dyslipidemia in a patient having type 2 diabetes, in a regimen which additionally comprises treatment with such as repaglinide.

In a further aspect the invention relates to a use of a GLP-1 agonist, preferably Arg³⁴, Lys²⁶(N^{ε}-(γ-Glu(N^{α}-hexadecanoyl)))-GLP-1(7-37), for the manufacture of a medicament for treating diabetes type 2 in a regimen which additionally comprises treatment with a thiazolidinedione such any one of troglitazone, ciglitazone, pioglitazone, rosiglitazone, or any one of the compounds disclosed in WO 97/41097, WO 97/41119, WO 97/41120, WO 00/41121 and WO 98/45292.

In a further aspect the invention relates to a use of a GLP-1 agonist, preferably Arg³⁴, Lys²⁶(N^{ε}-(γ-Glu(N^{α}-hexadecanoyl)))-GLP-1(7-37), for the manufacture of a medicament for treating dyslipidemia in a patient having type 2 diabetes, in a regimen which additionally comprises treatment with a thiazolidinedione such any one of troglitazone, ciglitazone, pioglitazone, rosiglitazone, or any one of the compounds disclosed in WO 97/41097, WO 97/41119, WO 97/41120, WO 00/41121 and WO 98/45292.

In a further aspect the invention relates to a use of a GLP-1 agonist, preferably Arg³⁴, Lys²⁶(N^{ε}-(γ-Glu(N^{α}-hexadecanoyl)))-GLP-1(7-37), for the manufacture of a medicament for treating diabetes type 1 in a regimen which additionally comprises treatment with an α-glucosidase inhibitor such as any one of miglitol or acarbose.

In a further aspect the invention relates to a use of a GLP-1 agonist, preferably Arg³⁴, Lys²⁶(N^{ε}-(γ-Glu(N^{α}-hexadecanoyl)))-GLP-1(7-37), for the manufacture of a medicament for treating diabetes type 2 in a regimen which additionally comprises treatment with an α-glucosidase inhibitor such as any one of miglitol or acarbose.

In a further aspect the invention relates to a use of a GLP-1 agonist, preferably Arg³⁴, Lys²⁶(N^{ε}-(γ-Glu(N^{α}-hexadecanoyl)))-GLP-1(7-37), for the manufacture of a medicament for treating dyslipidemia in a patient having type 2 diabetes, in a regimen which additionally comprises treatment with an α-glucosidase inhibitor such as any one of miglitol or acarbose.

In still another embodiment of the invention the present GLP-1 agonists may be administered in combination with any one of the insulin sensitizers disclosed in US 5,885,997, US 6,054,453, WO 99/19313, WO 00/50414, WO 00/63191, WO 00/63192, WO 00/63193, and WO 00/23425, WO 00/23415, WO 00/23451, WO 00/23445, WO 00/23417, WO 00/23416, WO 00/63153, WO 00/63196, WO 00/63209, WO 00/63190 and WO 00/63189. Preferred insulin sensitizers are selected from (-)-2-ethoxy-3-(4-(2-phenoxazin-10-yl-ethoxy)-phenyl)-propionic acid and 5-[[4-[3-Methyl-4-oxo-3,4-dihydro-2-quinazolinyl]methoxy]phenylmethyl]thiadiazolidine-2,4-dione.

In a further aspect the invention relates to a use of Arg³⁴, Lys²⁶(N^{ε}-(γ-Glu(N^{α-}hexadecanoyl)))-GLP-1 (7-37) for the manufacture of a medicament for treating diabetes type 1 in a regimen which additionally comprises treatment with (-)-2-ethoxy-3-(4-(2-phenoxazin-10-yl-ethoxy)-phenyl)-propionic acid.

In a further aspect the invention relates to a use of Arg³⁴, Lys²⁶(N^{ε}-(γ-Glu(N^{α-}hexadecanoyl)))-GLP-1 (7-37) for the manufacture of a medicament for treating diabetes type 2 in a regimen which additionally comprises treatment with (-)-2-ethoxy-3-(4-(2-phenoxazin-10-yl-ethoxy)-phenyl)-propionic acid.

In a further aspect the invention relates to a use of Arg³⁴, Lys²⁶(N^{ε}-(γ-Glu(N^{α-}hexadecanoyl)))-GLP-1 (7-37) for the manufacture of a medicament for treating dyslipidemia in a patient having type 2 diabetes, in a regimen which additionally comprises treatment with (-)-2-ethoxy-3-(4-(2-phenoxazin-10-yl-ethoxy)-phenyl)-propionic acid.

In a further aspect the invention relates to a use of Arg³⁴, Lys²⁶(N^{ε}-(γ-Glu(N^{α-}hexadecanoyl)))-GLP-1 (7-37) for the manufacture of a medicament for treating diabetes type 1 in a regimen which additionally comprises treatment with 5-[[4-[3-Methyl-4-oxo-3,4-dihydro-2-quinazolinyl]methoxy]phenyl-methyl]thiadiazolidine-2,4-dione.

In a further aspect the invention relates to a use of Arg³⁴, Lys²⁶(N^{ε}-(γ-Glu(N^{α-}hexadecanoyl)))-GLP-1 (7-37) for the manufacture of a medicament for treating diabetes type 2 in a regimen which additionally comprises treatment with 5-[[4-[3-Methyl-4-oxo-3,4-dihydro-2-quinazolinyl]methoxy]phenyl-methyl]thiadiazolidine-2,4-dione.

In a further aspect the invention relates to a use of Arg³⁴, Lys²⁶(N^{ε}-(γ-Glu(N^{α-}hexadecanoyl)))-GLP-1 (7-37) for the manufacture of a medicament for treating dyslipidemia in a patient having type 2 diabetes, in a regimen which additionally comprises treatment with 5-[[4-[3-Methyl-4-oxo-3,4-dihydro-2-quinazolinyl]methoxy]phenyl-methyl]thiadiazolidine-2,4-dione.

In yet another embodiment of the invention the present GLP-1 agonists, preferably Arg³⁴, Lys²⁶(N^{ε}-(γ-Glu(N^{α}-hexadecanoyl)))-GLP-1(7-37), may be administered in combination with nateglinide.

In still another embodiment of the invention the present GLP-1 agonists, preferably Arg³⁴, Lys²⁶(N^{ε}-(γ-Glu(N^{α}-hexadecanoyl)))-GLP-1(7-37), are administered in combination with an antihyperlipidemic agent or antilipidemic agent eg cholestyramine, colestipol, clofibrate, gemfibrozil, lovastatin, pravastatin, simvastatin, probucol or dextrothyroxine.

Furthermore, the present GLP-1 agonists, preferably Arg³⁴, Lys²⁶(N^{ε}-(γ-Glu(N^{α-}hexadecanoyl)))-GLP-1 (7-37), may be administered in combination with more than one of the above-mentioned compounds eg in combination with a sulphonylurea and metformin, a sulphonylurea and acarbose, repaglinide and metformin, insulin and a sulphonylurea, insulin and metformin, insulin and troglitazone, insulin and lovastatin, etc.

Furthermore, the GLP-1 agonists, preferably Arg³⁴, Lys²⁶(N^{ε}-(γ-Glu(N^{α-}hexadecanoyl)))-GLP-1 (7-37), according to the invention may be administered in combination with one or more antiobesity agents or appetite regulating agents. Such agents may be selected from the group consisting of CART (cocaine amphetamine regulated transcript) agonists, NPY (neuropeptide Y) antagonists, MC4 (melanocortin 4) agonists, orexin antagonists, TNF (tumor necrosis factor) agonists, CRF (corticotropin releasing factor) agonists, CRF BP (corticotropin releasing factor binding protein) antagonists, urocortin agonists, β3 agonists, MSH (melanocyte-stimulating hormone) agonists, MCH (melanocyte-concentrating hormone) antagonists, CCK (cholecystokinin) agonists, serotonin re-uptake inhibitors, serotonin and noradrenaline re-uptake inhibitors, 5HT (serotonin) agonists, bombesin agonists, galanin antagonists, growth hormone, growth hormone releasing compounds, TRH (thyreotropin releasing hormone) agonists, UCP 2 or 3 (uncoupling protein 2 or 3) modulators, leptin agonists, DA (dopamine) agonists (bromocriptin, doprexin), lipase/amylase inhibitors, PPAR modulators, RXR modulators or TR β agonists. In another embodiment of the invention the antiobesity agent is leptin. In another embodiment of the invention the antiobesity agent is dexamphetamine or amphetamine. In another embodiment of the invention the antiobesity agent is fenfluramine or dexfenfluramine. In still another embodiment of the invention the antiobesity agent is sibutramine. In a further embodiment of the invention the antiobesity agent is orlistat. In another embodiment of the invention the antiobesity agent is mazindol or phentermine.

Furthermore, the present GLP-1 agonists, preferably Arg³⁴, Lys²⁶(N^{ε}-(γ-Glu(N^{α-}hexadecanoyl)))-GLP-1 (7-37), may be administered in combination with one or more antihypertensive agents. Examples of antihypertensive agents are β-blockers such as alprenolol, atenolol, timolol, pindolol, propranolol and metoprolol, ACE (angiotensin converting enzyme) inhibitors such as benazepril, captopril, enalapril, fosinopril, lisinopril, quinapril and ramipril, calcium channel blockers such as nifedipine, felodipine, nicardipine, isradipine, nimodipine, diltiazem and verapamil, and α-blockers such as doxazosin, urapidil, prazosin and terazosin. Further reference can be made to Remington: The Science and Practice of Pharmacy, 19th Edition, Gennaro, Ed., Mack Publishing Co., Easton, PA, 1995.

It should be understood that any suitable combination of the GLP-1 agonists with one or more of the above-mentioned compounds and optionally one or more other pharmacologically active substances are considered to be within the scope of the present invention.

The present GLP-1 agonists, preferably Arg³⁴, Lys²⁶(N^{ε}-(γ-Glu(N^{α}-hexadecanoyl)))-GLP-1 (7-37), may also advantageously be combined with diet and/or exercise.

In one embodiment the GLP-1 agonist is GLP-1 (7-37) or GLP-1 (7-36) amide.

In a further embodiment of the invention the GLP-1 agonist is a GLP-1 analogue.

In a further embodiment of the invention the GLP-1 analogue is selected from the Thr⁸, Met⁸, Gly⁸ and Val⁸ analogues of GLP-1 (7-37) and GLP-1 (7-36) amide, more preferred the Gly⁸ and Val⁸ analogues of GLP-1 (7-37) and GLP-1 (7-36) amide, most preferred the Val⁸ analogues of GLP-1 (7-37) and GLP-1 (7-36) amide.

In a further embodiment of the invention the GLP-1 analogue has the formula II: 
7 8 9 10 11 12 13 14 15 16 17
His-Xaa-Xaa-Gly-Xaa-Phe-Thr-Xaa-Asp-Xaa-Xaa-

18 19 20 21 22 23 24 25 26 27 28
Xaa-Xaa-Xaa-Xaa-Xaa-Xaa-Xaa-Xaa-Xaa-Xaa-Phe-

29 30 31 32 33 34 35 36 37 38
Ile-Xaa-Xaa-Xaa-Xaa-Xaa-Xaa-Xaa-Xaa-Xaa

39 40 41 42 43 44 45
Xaa-Xaa-Xaa-Xaa-Xaa-Xaa-Xaa
(II)
 wherein
Xaa at position 8 is Ala, Gly, Ser, Thr, Leu, Ile, Val, Glu, Asp, Met, or Lys,
Xaa at position 9 is Glu, Asp, or Lys,
Xaa at position 11 is Thr, Ala, Gly, Ser, Leu, Ile, Val, Glu, Asp, or Lys,
Xaa at position 14 is Ser, Ala, Gly, Thr, Leu, Ile, Val, Glu, Asp, or Lys,
Xaa at position 16 is Val, Ala, Gly, Ser, Thr, Leu, Ile, Tyr, Glu, Asp, or Lys,
Xaa at position 17 is Ser, Ala, Gly, Thr, Leu, Ile, Val, Glu, Asp, or Lys,
Xaa at position 18 is Ser, Ala, Gly, Thr, Leu, Ile, Val, Glu, Asp, or Lys,
Xaa at position 19 is Tyr, Phe, Trp, Glu, Asp, or Lys,
Xaa at position 20 is Leu, Ala, Gly, Ser, Thr, Leu, Ile, Val, Glu, Asp, or Lys,
Xaa at position 21 is Glu, Asp, or Lys,
Xaa at position 22 is Gly, Ala, Ser, Thr, Leu, Ile, Val, Glu, Asp, or Lys,
Xaa at position 23 is Gin, Asn, Arg, Glu, Asp, or Lys,
Xaa at position 24 is Ala, Gly, Ser, Thr, Leu, Ile, Val, Arg, Glu, Asp, or Lys,
Xaa at position 25 is Ala, Gly, Ser, Thr, Leu, Ile, Val, Glu, Asp, or Lys,
Xaa at position 26 is Lys, Arg, Gln, Glu, Asp, or His,
Xaa at position 27 is Glu, Asp, or Lys,
Xaa at position 30 is Ala, Gly, Ser, Thr, Leu, Ile, Val, Glu, Asp, or Lys,
Xaa at position 31 is Trp, Phe, Tyr, Glu, Asp, or Lys,
Xaa at position 32 is Leu, Gly, Ala, Ser, Thr, Ile, Val, Glu, Asp, or Lys,
Xaa at position 33 is Val, Gly, Ala, Ser, Thr, Leu, Ile, Glu, Asp, or Lys,
Xaa at position 34 is Lys, Arg, Glu, Asp, or His,
Xaa at position 35 is Gly, Ala, Ser, Thr, Leu, Ile, Val, Glu, Asp, or Lys,
Xaa at position 36 is Arg, Lys, Glu, Asp, or His,
Xaa at position 37 is Gly, Ala, Ser, Thr, Leu, Ile, Val, Glu, Asp, or Lys, or is deleted,
Xaa at position 38 is Arg, Lys, Glu, Asp, or His, or is deleted,
Xaa at position 39 is Arg, Lys, Glu, Asp, or His, or is deleted,
Xaa at position 40 is Asp, Glu, or Lys, or is deleted,
Xaa at position 41 is Phe, Trp, Tyr, Glu, Asp, or Lys, or is deleted,
Xaa at position 42 is Pro, Lys, Glu, or Asp, or is deleted,
Xaa at position 43 is Glu, Asp, or Lys, or is deleted,
Xaa at position 44 is Glu, Asp, or Lys, or is deleted, and
Xaa at position 45 is Val, Glu, Asp, or Lys, or is deleted, or
(a) a C-1-6-ester thereof, (b) amide, C-1-6-alkylamide, or C-1-6-dialkylamide thereof and/or (c) a pharmaceutically acceptable salt thereof,
   provided that
   (i) when the amino acid at position 37, 38, 39, 40, 41, 42, 43 or 44 is deleted, then each amino acid downstream of the amino acid is also deleted.

In a further embodiment of the GLP-1 analogue of formula II, the amino acids at positions 37-45 are absent.

In another embodiment of the GLP-1 analogue of formula II, the amino acids at positions 38-45 are absent.

In another embodiment of the GLP-1 analogue of formula II, the amino acids at positions 39-45 are absent.

In another embodiment of the GLP-1 analogue of formula II, Xaa at position 8 is Ala, Gly, Ser, Thr, Met, or Val.

In another embodiment of the GLP-1 analogue of formula II, Xaa at position 8 is Gly, Thr, Met, or Val.

In another embodiment of the GLP-1 analogue of formula II, Xaa at position 8 is Val.

In another embodiment of the GLP-1 analogue of formula II, Xaa at position 9 is Glu.

In another embodiment of the GLP-1 analogue of formula II, Xaa at position 11 is Thr.

In another embodiment of the GLP-1 analogue of formula II, Xaa at position 14 is Ser.

In another embodiment of the GLP-1 analogue of formula II, Xaa at position 16 is Val.

In another embodiment of the GLP-1 analogue of formula II, Xaa at position 17 is Ser.

In another embodiment of the GLP-1 analogue of formula II, Xaa at position 18 is Ser, Lys, Glu, or Asp.

In another embodiment of the GLP-1 analogue of formula II, Xaa at position 19 is Tyr, Lys, Glu, or Asp.

In another embodiment of the GLP-1 analogue of formula II, Xaa at position 20 is Leu, Lys, Glu, or Asp.

In another embodiment of the GLP-1 analogue of formula II, Xaa at position 21 is Glu, Lys, or Asp.

In another embodiment of the GLP-1 analogue of formula II, Xaa at position 22 is Gly, Glu, Asp, or Lys.

In another embodiment of the GLP-1 analogue of formula II, Xaa at position 23 is Gln, Glu, Asp, or Lys.

In another embodiment of the GLP-1 analogue of formula II, Xaa at position 24 is Ala, Glu, Asp, or Lys.

In another embodiment of the GLP-1 analogue of formula II, Xaa at position 25 is Ala, Glu, Asp, or Lys.

In another embodiment of the GLP-1 analogue of formula II, Xaa at position 26 is Lys, Glu, Asp, or Arg.

In another embodiment of the GLP-1 analogue of formula II, Xaa at position 27 is Glu, Asp, or Lys.

In another embodiment of the GLP-1 analogue of formula II, Xaa at position 30 is Ala, Glu, Asp, or Lys.

In another embodiment of the GLP-1 analogue of formula II, Xaa at position 31 is Trp, Glu, Asp, or Lys.

In another embodiment of the GLP-1 analogue of formula II, Xaa at position 32 is Leu, Glu, Asp, or Lys.

In another embodiment of the GLP-1 analogue of formula II, Xaa at position 33 is Val, Glu, Asp, or Lys.

In another embodiment of the GLP-1 analogue of formula II, Xaa at position 34 is Lys, Arg, Glu, or Asp.

In another embodiment of the GLP-1 analogue of formula II, Xaa at position 35 is Gly, Glu, Asp, or Lys.

In another embodiment of the GLP-1 analogue of formula II, Xaa at position 36 is Arg, Lys, Glu, or Asp.

In another embodiment of the GLP-1 analogue of formula II, Xaa at position 37 is Gly, Glu, Asp, or Lys.

In another embodiment of the GLP-1 analogue of formula II, Xaa at position 38 is Arg, or Lys, or is deleted.

In another embodiment of the GLP-1 analogue of formula II, Xaa at position 39 is deleted.

In another embodiment of the GLP-1 analogue of formula II, Xaa at position 40 is deleted.

In another embodiment of the GLP-1 analogue of formula II, Xaa at position 41 is deleted.

In another embodiment of the GLP-1 analogue of formula II, Xaa at position 42 is deleted.

In another embodiment of the GLP-1 analogue of formula II, Xaa at position 43 is deleted.

In another embodiment of the GLP-1 analogue of formula II, Xaa at position 44 is deleted.

In another embodiment of the GLP-1 analogue of formula II, Xaa at position 45 is deleted.

In another embodiment of the GLP-1 analogue of formula II, Xaa at position 26 is Arg, each of Xaa at positions 37-45 is deleted, and each of the other Xaa is the amino acid in native GLP-1 (7-36).

In another embodiment of the GLP-1 analogue of formula II, Xaa at position 26 is Arg, each of Xaa at positions 38-45 is deleted, and each of the other Xaa is the amino acid in native GLP-1 (7-37).

In another embodiment of the GLP-1 analogue of formula II, Xaa at position 26 is Arg, each of Xaa at positions 39-45 is deleted, and each of the other Xaa is the amino acid in native GLP-1 (7-38).

In another embodiment of the GLP-1 analogue of formula II, Xaa at position 34 is Arg, each of Xaa at positions 37-45 is deleted, and each of the other Xaa is the amino acid in native GLP-1 (7-36).

In another embodiment of the GLP-1 analogue of formula II, Xaa at position 34 is Arg, each of Xaa at positions 38-45 is deleted, and each of the other Xaa is the amino acid in native GLP-1 (7-37).

In another embodiment of the GLP-1 analogue of formula II, Xaa at position 34 is Arg, each of Xaa at positions 39-45 is deleted, and each of the other Xaa is the amino acid in native GLP-1 (7-38).

In another embodiment of the GLP-1 analogue of formula II, Xaa at positions 26 and 34 is Arg, Xaa at position 36 is Lys, each of Xaa at positions 37-45 is deleted, and each of the other Xaa is the amino acid in native GLP-1 (7-36).

In another embodiment of the GLP-1 analogue of formula II, Xaa at positions 26 and 34 is Arg, Xaa at position 36 is Lys, each of Xaa at positions 38-45 is deleted, and each of the other Xaa is the amino acid in native GLP-1 (7-37).

In another embodiment of the GLP-1 analogue of formula II, Xaa at positions 26 and 34 is Arg, Xaa at position 36 is Lys, each of Xaa at positions 39-45 is deleted, and each of the other Xaa is the amino acid in native GLP-1 (7-38).

In another embodiment of the GLP-1 analogue of formula II, Xaa at positions 26 and 34 is Arg, Xaa at position 38 is Lys, each of Xaa at positions 39-45 is deleted, and each of the other Xaa is the amino acid in native GLP-1 (7-38).

In another embodiment of the GLP-1 analogue of formula II, Xaa at position 8 is Thr, Ser, Gly, or Val, Xaa at position 37 is Glu, Xaa at position 36 is Lys, each of Xaa at positions 38-45 is deleted, and each of the other Xaa is the amino acid in native GLP-1 (7-37).

In another embodiment of the GLP-1 analogue of formula II, Xaa at position 8 is Thr, Ser, Gly, or Val, Xaa at position 37 is Glu, Xaa at position 36 is Lys, each of Xaa at positions 39-45 is deleted, and each of the other Xaa is the amino acid in native GLP-1 (7-38).

In another embodiment of the GLP-1 analogue of formula II, Xaa at position 8 is Thr, Ser, Gly or Val, Xaa at position 37 is Glu, Xaa at position 38 is Lys, each of Xaa at positions 39-45 is deleted, and each of the other Xaa is the amino acid in native GLP-1 (7-38).

In another embodiment of the GLP-1 analogue of formula II, Xaa at position 18, 23 or 27 is Lys, and Xaa at positions 26 and 34 is Arg, each of Xaa at positions 37-45 is deleted, and each of the other Xaa is the amino acid in native GLP-1 (7-36).

In another embodiment of the GLP-1 analogue of formula II, Xaa at position 18, 23 or 27 is Lys, and Xaa at positions 26 and 34 is Arg, each of Xaa at positions 38-45 is deleted, and each of the other Xaa is the amino acid in native GLP-1 (7-37).

In another embodiment of the GLP-1 analogue of formula II, Xaa at position 18, 23 or 27 is Lys, and Xaa at positions 26 and 34 is Arg, each of Xaa at positions 39-45 is deleted, and each of the other Xaa is the amino acid in native GLP-1 (7-38).

In another embodiment of the GLP-1 analogue of formula II, Xaa at position 8 is Thr, Ser, Gly, or Val, Xaa at position 18, 23 or 27 is Lys, and Xaa at position 26 and 34 is Arg, each of Xaa at positions 37-45 is deleted, and each of the other Xaa is the amino acid in native GLP-1 (7-36).

In another embodiment of the GLP-1 analogue of formula II, Xaa at position 8 is Thr, Ser, Gly, or Val, Xaa at position 18, 23 or 27 is Lys, and Xaa at position 26 and 34 is Arg, each of Xaa at positions 38-45 is deleted, and each of the other Xaa is the amino acid in native GLP-1 (7-37).

In another embodiment of the GLP-1 analogue of formula II, Xaa at position 8 is Thr, Ser, Gly, or Val, Xaa at position 18, 23 or 27 is Lys, and Xaa at position 26 and 34 is Arg, each of Xaa at positions 39-45 is deleted, and each of the other Xaa is the amino acid in native GLP-1 (7-38).

Such GLP-1 analogues includes, but is not limited to, Arg²⁶-GLP-1(7-37); Arg³⁴-GLP-1 (7-37); Lys³⁶-GLP-1(7-37); Arg^{26,34}Lys³⁶-GLP-1(7-37); Arg^{26,34}Lys³⁸GLP-1(7-38); Arg^{26,34}Lys³⁹⁻GLP-1(7-39); Arg^{26,34}Lys⁴⁰-GLP-1(7-40); Arg²⁶Lys³⁶-GLP-1(7-37); Arg³⁴Lys³⁶-GLP-1(7-37); Arg²⁶Lys³⁹-GLP-1(7-39); Arg³⁴Lys⁴⁰-GLP-1(7-40); Arg^{26,34}Lys^{36,39}-GLP-1(7-39); Arg^{26,34}Lys^{36,40-}GLP-1 (7-40); Gly⁸Arg26-GLP-1(7-37); Gly⁸Arg³⁴-GLP-1(7-37); Val⁸-GLP-1(7-37); Thr⁸-GLP-1(7-37); Gly⁸-GLP-1(7-37); Met⁸-GLP-1(7-37); Gly⁸Lys³⁶-GLP-1(7-37); Gly⁸Arg^{26,34}Lys³⁶-GLP-1(7-37); Gly⁸Arg^{26,34}Lys³⁹-GLP-1(7-39); Gly⁸Arg^{26,34}Lys⁴⁰-GLP-1(7-40); Gly⁸Arg²⁶Lys³⁶-GLP-1(7-37); Gly⁸Arg³⁴Lys³⁶-GLP-1(7-37); Gly⁸Arg²⁶Lys³⁹-GLP-1(7-39); Gly⁸Arg³⁴Lys⁴⁰-GLP-1(7-40); Gly⁸Arg^{26,34}Lys^{36,39}-GLP-1(7-39); Gly⁸Arg^{26,34}Lys^{36,40}-GLP-1(7-40); Arg^{26,34}Lys³⁸GLP-1(7-38); Arg^{26,34}Lys³⁹GLP-1(7-39); Arg^{26,34}Lys⁴⁰GLP-1(7-40); Arg^{26,34}Lys⁴¹GLP-1(7-41); Arg^{26,34}Lys⁴²GLP-1(7-42); Arg^{26,34}Lys⁴³GLP-1(7-43); Arg^{26,34}Lys⁴⁴GLP-1(7-44); Arg^{26,34}Lys⁴⁵GLP-1(7-45); Arg^{26,34}Lys³⁸GLP-1(1-38); Arg^{26,34}Lys³⁹GLP-1(1-39); Arg^{26,34}Lys⁴⁰GLP-1(1-40); Arg^{26,34}Lys⁴¹GLP-1(1-41); Arg^{26,34}Lys⁴²GLP-1(1-42); Arg^{26,34}Lys⁴³GLP-1(1-43); Arg^{26,34}Lys⁴⁴GLP-1(1-44); Arg^{26,34}Lys⁴⁵GLP-1(1-45); Arg^{26,34}Lys³⁸GLP-1(2-38); Arg^{26,34}Lys³⁹GLP-1(2-39); Arg^{26,34}Lys⁴⁰GLP-1(2-40); Arg^{26,34}Lys⁴¹GLP-1(2-41); Arg^{26,34}Lys⁴²GLP-1(2-42); Arg^{26,34}Lys⁴³GLP-1(2-43); Arg^{26,34}Lys⁴⁴GLP-1(2-44); Arg^{26,34}Lys⁴⁵GLP-1(2-45); Arg^{26,34}Lys³⁸GLP-1(3-38); Arg^{26,34}Lys³⁹GLP-1(3-39); Arg^{26,34}Lys⁴⁰GLP-1(3-40); Arg^{26,34}Lys⁴¹GLP-1(3-41); Arg^{26,34}Lys⁴²GLP-1(3-42); Arg^{26,34}Lys⁴³GLP-1(3-43); Arg^{26,34}Lys⁴⁴GLP-1(3-44); Arg^{26,34}Lys⁴⁵GLP-1(3-45); Arg^{26,34}Lys³⁸GLP-1(4-38); Arg^{26,34}Lys³⁹GLP-1(4-39); Arg^{26,34}Lys⁴⁰GLP-1(4-40); Arg^{26,34}Lys⁴¹GLP-1(4-41); Arg^{26,34}Lys⁴²GLP-1(4-42); Arg^{26,34}Lys⁴³GLP-1(4-43); Arg^{26,34}Lys⁴⁴GLP-1(4-44); Arg^{26,34}Lys⁴⁵GLP-1(4-45); Arg^{26,34}Lys³⁸GLP-1(5-38); Arg^{26,34}Lys³⁹GLP-1(5-39); Arg^{26,34}Lys⁴⁰GLP-1(5-40); Arg^{26,34}Lys⁴¹GLP-1(5-41); Arg^{26,34}Lys⁴²GLP-1(5-42); Arg^{26,34}Lys⁴³GLP-1(5-43); Arg^{26,34}Lys⁴⁴GLP-1(5-44); Arg^{26,34}Lys⁴⁵GLP-1(5-45); Arg^{26,34}Lys³⁸GLP-1(6-38); Arg^{26,34}Lys³⁹GLP-1(6-39); Arg^{26,34}Lys⁴⁰GLP-1(6-40); Arg^{26,34}Lys⁴¹GLP-1(6-41); Arg^{26,34}Lys⁴²GLP-1(6-42); Arg^{26,34}Lys⁴³GLP-1(6-43); Arg^{26,34}Lys⁴⁴GLP-1(6-44); Arg^{26,34}Lys⁴⁵GLP-1(6-45); Arg²⁶Lys³⁸GLP-1(1-38); Arg³⁴Lys³⁸GLP-1(1-38); Arg^{26,34}Lys^{36,38}GLP-1(1-38); Arg²⁶Lys³⁸GLP-1(7-38); Arg³⁴Lys³⁸GLP-1(7-38); Arg^{26,34}Lys^{36,38}GLP-1(7-38); Arg^{26,34}Lys³⁸GLP-1(7-38); Arg²⁶Lys³⁹GLP-1(1-39); Arg³⁴Lys³⁹GLP-1(1-39); Arg^{26,34}Lys^{36,39}GLP-1(1-39); Arg²⁶Lys³⁹GLP-1(7-39); Arg³⁴Lys³⁹GLP-1(7-39) and Arg^{26,34}Lys^{36,39}GLP-1(7-39). Each one of these specific GLP-1 analogues constitutes an alternative embodiment of the invention.

In a still further embodiment of the invention the GLP-1 agonist is a GLP-1 derivative.

In a further embodiment of the invention the GLP-1 derivative has one or more lipophilic substituents attached to the parent peptide. The lipophilic substituents make the profile of action of the parent GLP-1 peptide more protracted, make the parent GLP-1 peptide more metabolically and physically stable, and/or increase the water solubility of the parent GLP-1 peptide.

The lipophilic substituent is characterised by having a solubility in water at 20°C in the range from about 0.1 mg/100 ml water to about 250 mg/100 ml water, preferable in the range from about 0.3 mg/100 ml water to about 75 mg/100 ml water. For instance, octanoic acid (C8) has a solubility in water at 20°C of 68 mg/100 ml, decanoic acid (C10) has a solubility in water at 20°C of 15 mg/100 ml, and octadecanoic acid (C18) has a solubility in water at 20°C of 0.3 mg/100 ml.

In a further embodiment of the invention the GLP-1 derivatives preferably have three lipophilic substituents, more preferably two lipophilic substituents, and most preferably one lipophilic substituent.

Each lipophilic substituent(s) preferably has 4-40 carbon atoms, more preferably 8-30 carbon atoms, even more preferably 8-25 carbon atoms, even more preferably 12-25 carbon atoms, and most preferably 14-18 carbon atoms.

The lipophilic substituent(s) contain a functional group which can be attached to one of the following functional groups of an amino acid of the parent GLP-1 peptide:
(a) the amino group attached to the alpha-carbon of the N-terminal amino acid,
(b) the carboxy group attached to the alpha-carbon of the C-terminal amino acid,
(c) the epsilon-amino group of any Lys residue,
(d) the carboxy group of the R group of any Asp and Glu residue,
(e) the hydroxy group of the R group of any Tyr, Ser and Thr residue,
(f) the amino group of the R group of any Trp, Asn, Gln, Arg, and His residue, or
(g) the thiol group of the R group of any Cys residue.

In an embodiment, a lipophilic substituent is attached to the carboxy group of the R group of any Asp and Glu residue.

In another embodiment, a lipophilic substituent is attached to the carboxy group attached to the alpha-carbon of the C-terminal amino acid.

In a most preferred embodiment, a lipophilic substituent is attached to the epsilon-amino group of any Lys residue.

Each lipophilic substituent contains a functional group which may be attached to a functional group of an amino acid of the parent GLP-1 peptide. For example, a lipophilic substituent may contain a carboxyl group which can be attached to an amino group of the parent GLP-1 peptide by means of an amide bond.

In an embodiment, the lipophilic substituent comprises a partially or completely hydrogenated cyclopentanophenathrene skeleton.

In another embodiment, the lipophilic substituent is a straight-chain or branched alkyl group.

In another embodiment, the lipophilic substituent is an acyl group of a straight-chain or branched fatty acid. Preferably, the lipophilic substituent is an acyl group having the formula CH₃(CH₂)ₙCO-, wherein n is an integer from 4 to 38, preferably an integer from 12 to 38, and most preferably is CH₃(CH₂)₁₂CO-, CH₃(CH₂)₁₄CO-, CH₃(CH₂)₁₆CO-, CH₃(CH₂)₁₈CO-, CH₃(CH₂)₂₀CO- and CH₃(CH₂)₂₂CO-. In a more preferred embodiment, the lipophilic substituent is tetradecanoyl. In a most preferred embodiment, the lipophilic substituent is hexadecanoyl.

In another embodiment of the present invention, the lipophilic substituent has a group which is negatively charged such as a carboxylic acid group. For example, the lipophilic substituent may be an acyl group of a straight-chain or branched alkane α,ω-dicarboxylic acid of the formula HOOC(CH₂)ₘCO-, wherein m is an integer from 4 to 38, preferably an integer from 12 to 38, and most preferably is HOOC(CH₂)₁₄CO-, HOOC(CH₂)₁₆CO-, HOOC(CH₂)₁₈CO-, HOOC(CH₂)₂₀CO- or HOOC(CH₂)₂₂CO-.

In a preferred embodiment of the invention, the lipophilic substituent is attached to the parent GLP-1 peptide by means of a spacer. A spacer must contain at least two functional groups, one to attach to a functional group of the lipophilic substituent and the other to a functional group of the parent GLP-1 peptide.

In an embodiment, the spacer is an amino acid residue except Cys or Met, or a dipeptide such as Gly-Lys. For purposes of the present invention, the phrase "a dipeptide such as Gly-Lys" means any combination of two amino acids except Cys or Met, preferably a dipeptide wherein the C-terminal amino acid residue is Lys, His or Trp, preferably Lys, and the N-terminal amino acid residue is Ala, Arg, Asp, Asn, Gly, Glu, Gln, Ile, Leu, Val, Phe, Pro, Ser, Tyr, Thr, Lys, His and Trp. Preferably, an amino group of the parent peptide forms an amide bond with a carboxylic group of the amino acid residue or dipeptide spacer, and an amino group of the amino acid residue or dipeptide spacer forms an amide bond with a carboxyl group of the lipophilic substituent.

Preferred spacers are lysyl, glutamyl, asparagyl, glycyl, beta-alanyl and gamma-aminobutanoyl, each of which constitutes an individual embodiment. Most preferred spacers are glutamyl and beta-alanyl. When the spacer is Lys, Glu or Asp, the carboxyl group thereof may form an amide bond with an amino group of the amino acid residue, and the amino group thereof may form an amide bond with a carboxyl group of the lipophilic substituent. When Lys is used as the spacer, a further spacer may in some instances be inserted between the ε-amino group of Lys and the lipophilic substituent. In one embodiment, such a further spacer is succinic acid which forms an amide bond with the ε-amino group of Lys and with an amino group present in the lipophilic substituent. In another embodiment such a further spacer is Glu or Asp which forms an amide bond with the ε-amino group of Lys and another amide bond with a carboxyl group present in the lipophilic substituent, that is, the lipophilic substituent is a N^{ε}-acylated lysine residue.

In another embodiment, the spacer is an unbranched alkane α,ω-dicarboxylic acid group having from 1 to 7 methylene groups, which spacer forms a bridge between an amino group of the parent peptide and an amino group of the lipophilic substituent. Preferably, the spacer is succinic acid.

In a further embodiment, the lipophilic substituent with the attached spacer is a group of the formula CH₃(CH₂)ₚNH-CO(CH₂)_{q}CO-, wherein p is an integer from 8 to 33, preferably from 12 to 28 and q is an integer from 1 to 6, preferably 2.

In a further embodiment, the lipophilic substituent with the attached spacer is a group of the formula CH₃(CH₂)ᵣCO-NHCH(COOH)(CH₂)₂CO-, wherein r is an integer from 4 to 24, preferably from 10 to 24.

In a further embodiment, the lipophilic substituent with the attached spacer is a group of the formula CH₃(CH₂)ₛCO-NHCH((CH₂)₂COOH)CO-, wherein s is an integer from 4 to 24, preferably from 10 to 24.

In a further embodiment, the lipophilic substituent is a group of the formula COOH(CH₂)ₜCO- wherein t is an integer from 6 to 24.

In a further embodiment, the lipophilic substituent with the attached spacer is a group of the formula -NHCH(COOH)(CH₂)₄NH-CO(CH₂)ᵤCH₃, wherein u is an integer from 8 to 18.

In a further embodiment, the lipophilic substituent with the attached spacer is a group of the formula CH₃(CH₂)ᵥCO-NH-(CH₂)_{z}-CO, wherein v is an integer from 4 to 24 and z is an integer from 1 to 6.

In a further embodiment, the lipophilic substituent with the attached spacer is a group of the formula -NHCH(COOH)(CH₂)₄NH-COCH((CH₂)₂COOH)NH-CO(CH₂)_{w}CH₃, wherein w is an integer from 10 to 16.

In a further embodiment, the lipophilic substituent with the attached spacer is a group of the formula -NHCH(COOH)(CH₂)₄NH-CO(CH₂)₂CH(COOH)NHCO(CH₂)ₓCH₃, wherein x is zero or an integer from 1 to 22, preferably 10 to 16.

In a further embodiment the GLP-1 derivative is derived from a GLP-1 fragment selected from the group comprising GLP-1 (7-35), GLP-1 (7-36), GLP-1 (7-36)amide, GLP-1 (7-37), GLP-1 (7-38), GLP-1 (7-39), GLP-1 (7-40) and GLP-1 (7-41) or an analogue thereof.

In a further embodiment of the GLP-1 derivative the designation analogue comprises derivatives wherein a total of up to fifteen, preferably up to ten amino acid residues have been exchanged with any α-amino acid residue.

In a further embodiment of the GLP-1 derivative the designation analogue comprises derivatives wherein a total of up to fifteen, preferably up to ten amino acid residues have been exchanged with any α-amino acid residue which can be coded for by the genetic code.

In a further embodiment of the GLP-1 derivative the designation analogue comprises derivatives wherein a total of up to six amino acid residues have been exchanged with another α-amino acid residue which can be coded for by the genetic code.

In a further embodiment the GLP-1 derivative is a GLP-1 derivative of formula I: 
7 8 9 10 11 12 13 14 15 16 17
His-Xaa-Xaa-Gly-Xaa-Phe-Thr-Xaa-Asp-Xaa-Xaa-

18 19 20 21 22 23 24 25 26 27 28
Xaa-Xaa-Xaa-Xaa-Xaa-Xaa-Xaa-Xaa-Xaa-Xaa-Phe-

29 30 31 32 33 34 35 36 37 38
Ile-Xaa-Xaa-Xaa-Xaa-Xaa-Xaa-Xaa-Xaa-Xaa

39 40 41 42 43 44 45
Xaa-Xaa-Xaa-Xaa-Xaa-Xaa-Xaa
(I)
 wherein
Xaa at position 8 is Ala, Gly, Ser, Thr, Leu, Ile, Val, Glu, Asp, Met, or Lys,
Xaa at position 9 is Glu, Asp, or Lys,
Xaa at position 11 is Thr, Ala, Gly, Ser, Leu, Ile, Val, Glu, Asp, or Lys,
Xaa at position 14 is Ser, Ala, Gly, Thr, Leu, Ile, Val, Glu, Asp, or Lys,
Xaa at position 16 is Val, Ala, Gly, Ser, Thr, Leu, Ile, Tyr, Glu, Asp, or Lys,
Xaa at position 17 is Ser, Ala, Gly, Thr, Leu, Ile, Val, Glu, Asp, or Lys,
Xaa at position 18 is Ser, Ala, Gly, Thr, Leu, Ile, Val, Glu, Asp, or Lys,
Xaa at position 19 is Tyr, Phe, Trp, Glu, Asp, or Lys,
Xaa at position 20 is Leu, Ala, Gly, Ser, Thr, Leu, Ile, Val, Glu, Asp, or Lys,
Xaa at position 21 is Glu, Asp, or Lys,
Xaa at position 22 is Gly, Ala, Ser, Thr, Leu, Ile, Val, Glu, Asp, or Lys,
Xaa at position 23 is Gln, Asn, Arg, Glu, Asp, or Lys,
Xaa at position 24 is Ala, Gly, Ser, Thr, Leu, Ile, Val, Arg, Glu, Asp, or Lys,
Xaa at position 25 is Ala, Gly, Ser, Thr, Leu, Ile, Val, Glu, Asp, or Lys,
Xaa at position 26 is Lys, Arg, Gln, Glu, Asp, or His,
Xaa at position 27 is Glu, Asp, or Lys,
Xaa at position 30 is Ala, Gly, Ser, Thr, Leu, Ile, Val, Glu, Asp, or Lys,
Xaa at position 31 is Trp, Phe, Tyr, Glu, Asp, or Lys,
Xaa at position 32 is Leu, Gly, Ala, Ser, Thr, Ile, Val, Glu, Asp, or Lys,
Xaa at position 33 is Val, Gly, Ala, Ser, Thr, Leu, Ile, Glu, Asp, or Lys,
Xaa at position 34 is Lys, Arg, Glu, Asp, or His,
Xaa at position 35 is Gly, Ala, Ser, Thr, Leu, Ile, Val, Glu, Asp, or Lys,
Xaa at position 36 is Arg, Lys, Glu, Asp, or His,
Xaa at position 37 is Gly, Ala, Ser, Thr, Leu, Ile, Val, Glu, Asp, or Lys, or is deleted,
Xaa at position 38 is Arg, Lys, Glu, Asp, or His, or is deleted,
Xaa at position 39 is Arg, Lys, Glu, Asp, or His, or is deleted,
Xaa at position 40 is Asp, Glu, or Lys, or is deleted,
Xaa at position 41 is Phe, Trp, Tyr, Glu, Asp, or Lys, or is deleted,
Xaa at position 42 is Pro, Lys, Glu, or Asp, or is deleted,
Xaa at position 43 is Glu, Asp, or Lys, or is deleted,
Xaa at position 44 is Glu, Asp, or Lys, or is deleted, and
Xaa at position 45 is Val, Glu, Asp, or Lys, or is deleted, or
(a) a C-1-6-ester thereof, (b) amide, C-1-6-alkylamide, or C-1-6-dialkylamide thereof and/or (c) a pharmaceutically acceptable salt thereof,
   provided that
   (i) when the amino acid at position 37, 38, 39, 40, 41, 42, 43 or 44 is deleted, then each amino acid downstream of the amino acid is also deleted,
   (ii) the derivative of the GLP-1 analog contains only one or two Lys,
   (iii) the ε-amino group of one or both Lys is substituted with a lipophilic substituent optionally via a spacer.
      In a further embodiment of the GLP-1 derivative of formula I, the amino acids at positions 37-45 are absent.

In another embodiment of the GLP-1 derivative of formula I, the amino acids at positions 38-45 are absent.

In another embodiment of the GLP-1 derivative of formula I, the amino acids at positions 39-45 are absent.

In another embodiment of the GLP-1 derivative of formula I, Xaa at position 8 is Ala, Gly, Ser, Thr, Leu, Ile, Val, Glu, Asp, or Lys,

In another embodiment of the GLP-1 derivative of formula I, Xaa at position 8 is Ala, Gly, Ser, Thr, or Val.

In another embodiment of the GLP-1 derivative of formula I, Xaa at position 9 is Glu.

In another embodiment of the GLP-1 derivative of formula I, Xaa at position 11 is Thr.

In another embodiment of the GLP-1 derivative of formula I, Xaa at position 14 is Ser.

In another embodiment of the GLP-1 derivative of formula I, Xaa at position 16 is Val.

In another embodiment of the GLP-1 derivative of formula I, Xaa at position 17 is Ser.

In another embodiment of the GLP-1 derivative of formula I, Xaa at position 18 is Ser, Lys, Glu, or Asp.

In another embodiment of the GLP-1 derivative of formula I, Xaa at position 19 is Tyr, Lys, Glu, or Asp.

In another embodiment of the GLP-1 derivative of formula I, Xaa at position 20 is Leu, Lys, Glu, or Asp.

In another embodiment of the GLP-1 derivative of formula I, Xaa at position 21 is Glu, Lys, or Asp.

In another embodiment of the GLP-1 derivative of formula I, Xaa at position 22 is Gly, Glu, Asp, or Lys.

In another embodiment of the GLP-1 derivative of formula I, Xaa at position 23 is Gln, Glu, Asp, or Lys.

In another embodiment of the GLP-1 derivative of formula I, Xaa at position 24 is Ala, Glu, Asp, or Lys.

In another embodiment of the GLP-1 derivative of formula I, Xaa at position 25 is Ala, Glu, Asp, or Lys.

In another embodiment of the GLP-1 derivative of formula I, Xaa at position 26 is Lys, Glu, Asp, or Arg.

In another embodiment of the GLP-1 derivative of formula I, Xaa at position 27 is Glu, Asp, or Lys.

In another embodiment of the GLP-1 derivative of formula I, Xaa at position 30 is Ala, Glu, Asp, or Lys.

In another embodiment of the GLP-1 derivative of formula I, Xaa at position 31 is Trp, Glu, Asp, or Lys.

In another embodiment of the GLP-1 derivative of formula I, Xaa at position 32 is Leu, Glu, Asp, or Lys.

In another embodiment of the GLP-1 derivative of formula I, Xaa at position 33 is Val, Glu, Asp, or Lys.

In another embodiment of the GLP-1 derivative of formula I, Xaa at position 34 is Lys, Arg, Glu, or Asp.

In another embodiment of the GLP-1 derivative of formula I, Xaa at position 35 is Gly, Glu, Asp, or Lys.

In another embodiment of the GLP-1 derivative of formula I, Xaa at position 36 is Arg, Lys, Glu, or Asp.

In another embodiment of the GLP-1 derivative of formula I, Xaa at position 37 is Gly, Glu, Asp, or Lys.

In another embodiment of the GLP-1 derivative of formula I, Xaa at position 38 is Arg, or Lys, or is deleted.

In another embodiment of the GLP-1 derivative of formula I, Xaa at position 39 is deleted.

In another embodiment of the GLP-1 derivative of formula I, Xaa at position 40 is deleted.

In another embodiment of the GLP-1 derivative of formula I, Xaa at position 41 is deleted.

In another embodiment of the GLP-1 derivative of formula I, Xaa at position 42 is deleted.

In another embodiment of the GLP-1 derivative of formula I, Xaa at position 43 is deleted.

In another embodiment of the GLP-1 derivative of formula I, Xaa at position 44 is deleted.

In another embodiment of the GLP-1 derivative of formula I, Xaa at position 45 is deleted.

In another embodiment of the GLP-1 derivative of formula I, Xaa at position 26 is Arg, each of Xaa at positions 37-45 is deleted, and each of the other Xaa is the amino acid in native GLP-1 (7-36).

In another embodiment of the GLP-1 derivative of formula I, Xaa at position 26 is Arg, each of Xaa at positions 38-45 is deleted, and each of the other Xaa is the amino acid in native GLP-1 (7-37).

In another embodiment of the GLP-1 derivative of formula I, Xaa at position 26 is Arg, each of Xaa at positions 39-45 is deleted, and each of the other Xaa is the amino acid in native GLP-1 (7-38).

In another embodiment of the GLP-1 derivative of formula I, Xaa at position 34 is Arg, each of Xaa at positions 37-45 is deleted, and each of the other Xaa is the amino acid in native GLP-1 (7-36).

In another embodiment of the GLP-1 derivative of formula I, Xaa at position 34 is Arg, each of Xaa at positions 38-45 is deleted, and each of the other Xaa is the amino acid in native GLP-1 (7-37).

In another embodiment of the GLP-1 derivative of formula I, Xaa at position 34 is Arg, each of Xaa at positions 39-45 is deleted, and each of the other Xaa is the amino acid in native GLP-1 (7-38).

In another embodiment of the GLP-1 derivative of formula I, Xaa at positions 26 and 34 is Arg, Xaa at position 36 is Lys, each of Xaa at positions 37-45 is deleted, and each of the other Xaa is the amino acid in native GLP-1 (7-36).

In another embodiment of the GLP-1 derivative of formula I, Xaa at positions 26 and 34 is Arg, Xaa at position 36 is Lys, each of Xaa at positions 38-45 is deleted, and each of the other Xaa is the amino acid in native GLP-1 (7-37).

In another embodiment of the GLP-1 derivative of formula I, Xaa at positions 26 and 34 is Arg, Xaa at position 36 is Lys, each of Xaa at positions 39-45 is deleted, and each of the other Xaa is the amino acid in native GLP-1 (7-38).

In another embodiment of the GLP-1 derivative of formula I, Xaa at positions 26 and 34 is Arg, Xaa at position 38 is Lys, each of Xaa at positions 39-45 is deleted, and each of the other Xaa is the amino acid in native GLP-1 (7-38).

In another embodiment of the GLP-1 derivative of formula I, Xaa at position 8 is Thr, Ser, Gly or Val, Xaa at position 37 is Glu, Xaa at position 36 is Lys, each of Xaa at positions 38-45 is deleted, and each of the other Xaa is the amino acid in native GLP-1 (7-37).

In another embodiment of the GLP-1 derivative of formula I, Xaa at position 8 is Thr, Ser, Gly or Val, Xaa at position 37 is Glu, Xaa at position 36 is Lys, each of Xaa at positions 39-45 is deleted, and each of the other Xaa is the amino acid in native GLP-1 (7-38).

In another embodiment of the GLP-1 derivative of formula I, Xaa at position 8 is Thr, Ser, Gly or Val, Xaa at position 37 is Glu, Xaa at position 38 is Lys, each of Xaa at positions 39-45 is deleted, and each of the other Xaa is the amino acid in native GLP-1 (7-38).

In another embodiment of the GLP-1 derivative of formula I, Xaa at position 18, 23 or 27 is Lys, and Xaa at positions 26 and 34 is Arg, each of Xaa at positions 37-45 is deleted, and each of the other Xaa is the amino acid in native GLP-1 (7-36).

In another embodiment of the GLP-1 derivative of formula I, Xaa at position 18, 23 or 27 is Lys, and Xaa at positions 26 and 34 is Arg, each of Xaa at positions 38-45 is deleted, and each of the other Xaa is the amino acid in native GLP-1 (7-37).

In another embodiment of the GLP-1 derivative of formula I, Xaa at position 18, 23 or 27 is Lys, and Xaa at positions 26 and 34 is Arg, each of Xaa at positions 39-45 is deleted, and each of the other Xaa is the amino acid in native GLP-1 (7-38).

In another embodiment of the GLP-1 derivative of formula I, Xaa at position 8 is Thr, Ser, Gly, or Val, Xaa at position 18, 23 or 27 is Lys, and Xaa at position 26 and 34 is Arg, each of Xaa at positions 37-45 is deleted, and each of the other Xaa is the amino acid in native GLP-1 (7-36).

In another embodiment of the GLP-1 derivative of formula I, Xaa at position 8 is Thr, Ser, Gly, or Val, Xaa at position 18, 23 or 27 is Lys, and Xaa at position 26 and 34 is Arg, each of Xaa at positions 38-45 is deleted, and each of the other Xaa is the amino acid in native GLP-1 (7-37).

In another embodiment of the GLP-1 derivative of formula I, Xaa at position 8 is Thr, Ser, Gly, or Val, Xaa at position 18, 23 or 27 is Lys, and Xaa at position 26 and 34 is Arg, each of Xaa at positions 39-45 is deleted, and each of the other Xaa is the amino acid in native GLP-1 (7-38).

Such GLP-1 derivatives includes, but is not limited to,
Lys³⁴ (N^{ε}-(γ-glutamyl(N^{α}-tetradecanoyl))) GLP-1 (7-37),
Arg^{26,34},Lys⁸(N^{ε}-(γ-glutamyl(N^{α}-hexadecanoyl))) GLP-1 (7-37),
Arg³⁴,Lys²⁶(N^{ε}-(γ-glutamyl(N^{α}-dodecanoyl))) GLP-1 (7-37),
Arg³⁴,Lys²⁶(N^{ε}-(β-alanyl(N^{α}-hexadecanoyl))) GLP-1 (7-37),
Arg³⁴,Lys²⁶(N^{ε}-(α-glutamyl(N^{α}-hexadecanoyl))) GLP-1 (7-37),
Arg³⁴,Lys²⁶(N^{ε}-(piperidinyl-4-carbonyl(N-hexadecanoyl))) GLP-1 (7-37),
Arg³⁴,Lys²⁶(N^{ε}-(γ-glutamyl(N^{α}-decanoyl))) GLP-1 (7-37),
Glu^{22,23,30}Arg^{26,34}Lys³⁸(N^{ε}-(γ-glutamyl(N^{α}-tetradecanoyl)))-GLP-1(7-38)-OH,
Glu^{23,26}Arg³⁴Lys³⁸(N^{ε}-(γ-glutamyl(N^{α}-tetradecanoyl)))-GLP-1(7-38)-OH,
Lys^{26,34}-bis(N^{ε}-(γ-glutamyl(N^{α}-tetradecanoyl)))-GLP-1(7-37)-OH,
Lys^{26,34}-bis(N^{ε}-(γ-glutamyl(N^{α}-hexadecanoyl)))-GLP-1(7-37)-OH,
Arg³⁴Lys²⁶(N^{ε}-(γ-glutamyl(N^{α}-hexadecanoyl)))-GLP-1(7-37)-OH,
Arg^{26,34}Lys³⁸(N^{ε}-(γ-glutamyl(N^{α}-tetradecanoyl)))-GLP-1(7-38)-OH,
Arg^{26,34}Lys³⁸(N^{ε}-(γ-glutamyl(N^{α}-hexadecanoyl)))-GLP-1(7-38)-OH,
Arg³⁴Lys²⁶(N^{ε}-(γ-glutamyl(N^{α}-tetradecanoyl)))-GLP-1(7-37)-OH,
Arg^{26,34}Lys³⁸(N^{ε}-(γ-glutamyl(N^{α}-octadecanoyl)))-GLP-1(7-38)-OH.
Glu^{22,23,30}Arg^{26,34}Lys³⁸(N^{ε}-(γ-glutamyl(N^{α}-tetradecanoyl)))-GLP-1(7-38)-OH,
Glu^{23,26}Arg³⁴Lys³⁸(N^{ε}-(γ-glutamyl(N^{α}-tetradecanoyl)))-GLP-1(7-38)-OH,
Lys^{26,34}-bis(N^{ε}-(ω-carboxytridecanoyl))-GLP-1(7-37)-OH,
Lys^{26,34}-bis(N^{ε}-(γ-glutamyl(N^{α}-tetradecanoyl)))-GLP-1(7-37)-OH,
Arg^{26,34}Lys³⁸(N^{ε}-(ω-carboxypentadecanoyl))-GLP-1(7-38)-OH,
Lys^{26,34}-bis(N^{ε}-(γ-glutamyl(N^{α}-hexadecanoyl)))-GLP-1(7-37)-OH,
Arg³⁴Lys²⁶(N^{ε}-(γ-glutamyl(N^{α}-hexadecanoyl)))-GLP-1(7-37)-OH,
Arg^{26,34}Lys³⁸(N^{ε}-(γ-glutamyl(N^{α}-tetradecanoyl)))-GLP-1(7-38)-OH,
Arg^{26,34}Lys³⁸(N^{ε}-(ω-carboxypentadecanoyl))-GLP-1(7-38)-OH,
Arg^{26,34}Lys³⁸(N^{ε}-(γ-glutamyl(N^{α}-hexadecanoyl)))-GLP-1(7-38)-OH,
Arg^{18,23,26,30,34}Lys³⁸(N^{ε}-hexadecanoyl)-GLP-1(7-38)-OH,
Arg^{26,34}Lys³⁸(N^{ε}-(ω-carboxytridecanoyl))-GLP-1(7-38)-OH,
Arg³⁴Lys²⁶(N^{ε}-(γ-glutamyl(N^{α}-tetradecanoyl)))-GLP-1(7-37)-OH,
Arg^{26,34}Lys³⁸(N^{ε}-(γ-glutamyl(N^{α}-octadecanoyl)))-GLP-1(7-38)-OH,
Glu^{22,23,30}Arg^{26,34}Lys³⁸(N^{ε}-(β-alanyl(N^{α}-tetradecanoyl)))-GLP-1(7-38)-OH,
Glu^{23,26}Arg³⁴Lys³⁸(N^{ε}-(β-alanyl(N^{α}-tetradecanoyl)))-GLP-1(7-38)-OH,
Lys^{26,34}-bis(N^{ε}-(β-alanyl(N^{α}-tetradecanoyl)))-GLP-1(7-37)-OH,
Lys^{26,34}-bis(N^{ε}-(β-alanyl(N^{α}-hexadecanoyl)))-GLP-1(7-37)-OH,
Arg³⁴Lys²⁶(N^{ε}-(β-alanyl(N^{α}-hexadecanoyl)))-GLP-1(7-37)-OH,
Arg^{26,34}Lys³⁸(N^{ε}-(β-alanyl(N^{α}-tetradecanoyl)))-GLP-1(7-38)-OH,
Arg^{26,34}Lys³⁸(N^{ε}-(β-alanyl(N^{α}-hexadecanoyl)))-GLP-1(7-38)-OH,
Arg³⁴Lys²⁶(N^{ε}-(β-alanyl(N^{α}-tetradecanoyl)))-GLP-1(7-37)-OH,
Arg^{26,34}Lys³⁸(N^{ε}-(β-alanyl(N^{α}-octadecanoyl)))-GLP-1(7-38)-OH.
Glu^{22,23,30}Arg^{26,34}Lys³⁸(N^{ε}-(β-alanyl(N^{α}-tetradecanoyl)))-GLP-1(7-38)-OH,
Glu^{23,26}Arg³⁴Lys³⁸(N^{ε}-(β-alanyl(N^{α}-tetradecanoyl)))-GLP-1(7-38)-OH,
Lys^{26,34}-bis(N^{ε}-(β-alanyl(N^{α}-tetradecanoyl)))-GLP-1(7-37)-OH,
Lys^{26,34}-bis(N^{ε}-(β-alanyl(N^{α}-hexadecanoyl)))-GLP-1(7-37)-OH,
Arg³⁴Lys²⁶(N^{ε}-(β-alanyl(N^{α}-hexadecanoyl)))-GLP-1(7-37)-OH,
Arg^{26,34}Lys³⁸(N^{ε}-(β-alanyl(N^{α}-tetradecanoyl)))-GLP-1(7-38)-OH,
Arg^{26,34}Lys³⁸(N^{ε}-(β-alanyl(N^{α}-hexadecanoyl)))-GLP-1(7-38)-OH,
Arg³⁴Lys²⁶(N^{ε}-(β-alanyl(N^{α}-tetradecanoyl)))-GLP-1(7-37)-OH,
Arg^{26,34}Lys³⁸(N^{ε}-(β-alanyl(N^{α}-octadecanoyl)))-GLP-1(7-38)-OH,
Lys²⁶(N^{ε}-tetradecanoyl)-GLP-1(7-37);
Lys³⁴(N^{ε}-tetradecanoyl)-GLP-1(7-37);
Lys^{26,34}-bis(N^{ε}-tetradecanoyl)-GLP-1(7-37);
Gly⁸Lys²⁶(N^{ε}-tetradecanoyl)-GLP-1(7-37);
Gly⁸Lys³⁴(N^{ε}-tetradecanoyl)-GLP-1(7-37);
Gly⁸Lys^{26,34}-bis(N^{ε}-tetradecanoyl)-GLP-1(7-37);
Val⁸Lys²⁶(N^{ε}-tetradecanoyl)-GLP-1(7-37);
Val⁸Lys³⁴(N^{ε}-tetradecanoyl)-GLP-1(7-37);
Val⁸Lys^{26,34}-bis(N^{ε}-tetradecanoyl)-GLP-1(7-37);
Arg²⁶Lys³⁴(N^{ε}-tetradecanoyl)-GLP-1(7-37);
Lys²⁶(N^{ε}-tetradecanoyl)-GLP-1(7-38);
Lys³⁴(N^{ε}-tetradecanoyl)-GLP-1(7-38);
Lys^{26,34}-bis(N^{ε}-tetradecanoyl)-GLP-1(7-38);
Gly⁸Lys²⁶(N^{ε}-tetradecanoyl)-GLP-1(7-38);
Gly⁸Lys³⁴(N^{ε}-tetradecanoyl)-GLP-1(7-38);
Gly⁸Lys^{26,34}-bis(N^{ε}-tetradecanoyl)-GLP-1(7-38);
Arg²⁶Lys³⁴(N^{ε}-tetradecanoyl)-GLP-1(7-38);
Lys²⁶(N^{ε}-tetradecanoyl)-GLP-1(7-39);
Lys³⁴(N^{ε}-tetradecanoyl)-GLP-1(7-39);
Lys^{26,34}-bis(N^{ε}-tetradecanoyl)-GLP-1(7-39);
Gly⁸Lys²⁶(N^{ε}-tetradecanoyl)-GLP-1(7-39);
Gly⁸Lys³⁴(N^{ε}-tetradecanoyl)-GLP-1(7-39);
Gly⁸Lys^{26,34}-bis(N^{ε}-tetradecanoyl)-GLP-1(7-39);
Arg²⁶Lys³⁴(N^{ε}-tetradecanoyl)-GLP-1(7-39);
Lys²⁶(N^{ε}-tetradecanoyl)-GLP-1(7-40);
Lys³⁴(N^{ε}-tetradecanoyl)-GLP-1(7-40);
Lys^{26,34}-bis(N^{ε}-tetradecanoyl)-GLP-1(7-40);
Gly⁸Lys²⁶(N^{ε}-tetradecanoyl)-GLP-1(7-40);
Gly⁸Lys³⁴(N^{ε}-tetradecanoyl)-GLP-1(7-40);
Gly⁸Lys^{26,34}-bis(N^{ε}-tetradecanoyl)-GLP-1(7-40);
Arg²⁶Lys³⁴(N^{ε}-tetradecanoyl)-GLP-1(7-40);
Lys²⁶(N^{ε}-tetradecanoyl)-GLP-1(7-36);
Lys³⁴(N^{ε}-tetradecanoyl)-GLP-1(7-36);
Lys^{26,34}-bis(N^{ε}-tetradecanoyl)-GLP-1(7-36);
Gly⁸Lys²⁶(N^{ε}-tetradecanoyl)-GLP-1(7-36);
Gly⁸Lys³⁴(N^{ε}-tetradecanoyl)-GLP-1(7-36);
Gly⁸Lys^{26,34}-bis(N^{ε}-tetradecanoyl)-GLP-1(7-36);
Arg²⁶Lys³⁴(N^{ε}-tetradecanoyl)-GLP-1(7-36);
Lys²⁶(N^{ε}-tetradecanoyl)-GLP-1(7-35);
Lys³⁴(N^{ε}-tetradecanoyl)-GLP-1(7-35);
Lys^{26,34}-bis(N^{ε}-tetradecanoyl)-GLP-1(7-35);
Gly⁸Lys²⁶(N^{ε}-tetradecanoyl)-GLP-1(7-35);
Gly⁸Lys³⁴(N^{ε}-tetradecanoyl)-GLP-1(7-35);
Gly⁸Lys^{26,34}-bis(N^{ε}-tetradecanoyl)-GLP-1(7-35);
Arg²⁶Lys³⁴(N^{ε}-tetradecanoyl)-GLP-1(7-35);
Lys²⁶(N^{ε}-tetradecanoyl)-GLP-1(7-36)amide;
Lys³⁴(N^{ε}-tetradecanoyl)-GLP-1(7-36)amide;
Lys^{26,34}-bis(N^{ε}-tetradecanoyl)-GLP-1(7-36)amide;
Gly⁸Lys²⁶(N^{ε}-tetradecanoyl)-GLP-1(7-36)amide;
Gly⁸Lys³⁴(N^{ε}-tetradecanoyl)-GLP-1(7-36)amide;
Gly⁸Lys^{26,34}-bis(N^{ε}-tetradecanoyl)-GLP-1(7-36)amide;
Arg²⁶Lys³⁴(N^{ε}-tetradecanoyl)-GLP-1(7-36)amide;
Gly⁸Arg²⁶Lys³⁴(N^{ε}-tetradecanoyl)-GLP-1(7-37);
Lys²⁶(N^{ε}-tetradecanoyl)Arg³⁴-GLP-1 (7-37);
Gly⁸Lys²⁶(N^{ε}-tetradecanoyl)Arg³⁴-GLP-1(7-37);
Arg^{26,34}Lys³⁶(N^{ε}-tetradecanoyl)-GLP-1(7-37);
Gly⁸Arg^{26,34}Lys³⁶(N^{ε}-tetradecanoyl)-GLP-1(7-37);
Gly⁸Arg²⁶Lys³⁴(N^{ε}-tetradecanoyl)-GLP-1(7-38);
Lys²⁶(N^{ε}-tetradecanoyl)Arg³⁴-GLP-1(7-38);
Gly⁸Lys²⁶(N^{ε}-tetradecanoyl)Arg³⁴-GLP-1(7-38);
Arg^{26,34}Lys³⁶(N^{ε}-tetradecanoyl)-GLP-1(7-38);
Arg^{26,34}Lys³⁸(N^{ε}-tetradecanoyl)-GLP-1(7-38);
Gly⁸Arg^{26,34}Lys³⁶(N^{ε}-tetradecanoyl)-GLP-1(7-38);
Gly⁸Arg²⁶Lys³⁴(N^{ε}-tetradecanoyl)-GLP-1(7-39);
Lys²⁶(N^{ε}-tetradecanoyl)Arg³⁴-GLP-1(7-39);
Gly⁸Lys²⁶(N^{ε}-tetradecanoyl)Arg³⁴-GLP-1(7-39);
Arg^{26,34}Lys³⁶(N^{ε}-tetradecanoyl)-GLP-1(7-39);
Gly⁸Arg^{26,34}Lys³⁶(N^{ε}-tetradecanoyl)-GLP-1(7-39);
Gly⁸Arg²⁶Lys³⁴(N^{ε}-tetradecanoyl)-GLP-1(7-40);
Lys²⁶(N^{ε}-tetradecanoyl)Arg³⁴-GLP-1(7-40);
Gly⁸Lys²⁶(N^{ε}-tetradecanoyl)Arg³⁴-GLP-1(7-40);
Arg^{26,34}Lys³⁶(N^{ε}-tetradecanoyl)-GLP-1(7-40);
Gly⁸Arg^{26,34}Lys³⁶(N^{ε}-tetradecanoyl)-GLP-1(7-40);
Lys²⁶(N^{ε}-(ω-carboxynonadecanoyl))-GLP-1(7-37);
Lys³⁴(N^{ε}-(ω-carboxynonadecanoyl))-GLP-1(7-37);
Lys^{26,34}-bis(N^{ε}-(ω-carboxynonadecanoyl))-GLP-1(7-37);
Gly⁸Lys²⁶(N^{ε}-(ω-carboxynonadecanoyl))-GLP-1(7-37);
Gly⁸Lys³⁴(N^{ε}-(ω-carboxynonadecanoyl))-GLP-1(7-37);
Gly⁸Lys^{26,34}-bis(N^{ε}-(ω-carboxynonadecanoyl))-GLP-1(7-37);
Lys²⁶(N^{ε}-(ω-carboxynonadecanoyl))-GLP-1(7-38);
Lys³⁴(N^{ε}-(ω-carboxynonadecanoyl))-GLP-1(7-38);
Lys^{26,34}-bis(N^{ε}-(ω-carboxynonadecanoyl))-GLP-1(7-38);
Gly⁸Lys²⁶(N^{ε}-(ω-carboxynonadecanoyl))-GLP-1(7-38);
Gly⁸Lys³⁴(N^{ε}-(ω-carboxynonadecanoyl))-GLP-1(7-38);
Gly⁸Lys^{26,34}-bis(N^{ε}-(ω-carboxynonadecanoyl))-GLP-1(7-38);
Lys²⁶(N^{ε}-(ω-carboxynonadecanoyl))-GLP-1(7-39);
Lys³⁴(N^{ε}-(ω-carboxynonadecanoyl))-GLP-1(7-39);
Lys^{26,34}-bis(N^{ε}-(ω-carboxynonadecanoyl))-GLP-1(7-39);
Gly⁸Lys²⁶(N^{ε}-(ω-carboxynonadecanoyl))-GLP-1(7-39);
Gly⁸Lys³⁴(N^{ε}-(ω-carboxynonadecanoyl))-GLP-1(7-39);
Gly⁸Lys^{26,34}-bis(N^{ε}-(ω-carboxynonadecanoyl))-GLP-1(7-39);
Lys²⁶(N^{ε}-(ω-carboxynonadecanoyl))-GLP-1(7-40);
Lys³⁴(N^{ε}-(ω-carboxynonadecanoyl))-GLP-1(7-40);
Lys^{26,34}-bis(N^{ε}-(ω-carboxynonadecanoyl))-GLP-1(7-40);
Gly⁸Lys²⁶(N^{ε}-(ω-carboxynonadecanoyl))-GLP-1(7-40);
Gly⁸Lys³⁴(N^{ε}-(ω-carboxynonadecanoyl))-GLP-1(7-40);
Gly⁸Lys^{26,34}-bis(N^{ε}-(ω-carboxynonadecanoyl))-GLP-1(7-40);
Lys²⁶(N^{ε}-(ω-carboxynonadecanoyl))-GLP-1(7-36);
Lys³⁴(N^{ε}-(ω-carboxynonadecanoyl))-GLP-1(7-36);
Lys^{26,34}-bis(N^{ε}-(ω-carboxynonadecanoyl))-GLP-1(7-36);
Gly⁸Lys²⁶(N^{ε}-(ω-carboxynonadecanoyl))-GLP-1(7-36);
Gly⁸Lys³⁴(N^{ε}-(ω-carboxynonadecanoyl))-GLP-1(7-36);
Gly⁸Lys^{26,34}-bis(N^{ε}-(ω-carboxynonadecanoyl))-GLP-1(7-36);
Lys²⁶(N^{ε}-(ω-carboxynonadecanoyl))-GLP-1(7-36)amide;
Lys³⁴(N^{ε}-(ω-carboxynonadecanoyl))-GLP-1(7-36)amide;
Lys^{26,34}-bis(N^{ε}-(ω-carboxynonadecanoyl))-GLP-1(7-36)amide;
Gly⁸Lys²⁶(N^{ε}-(ω-carboxynonadecanoyl))-GLP-1(7-36)amide;
Gly⁸Lys³⁴(N^{ε}-(ω-carboxynonadecanoyl))-GLP-1(7-36)amide;
Gly⁸Lys^{26,34}-bis(N^{ε}-(ω-carboxynonadecanoyl))-GLP-1(7-36)amide;
Lys²⁶(N^{ε}-(ω-carboxynonadecanoyl))-GLP-1(7-35);
Lys³⁴(N^{ε}-(ω-carboxynonadecanoyl))-GLP-1(7-35);
Lys^{26,34}-bis(N^{ε}-(ω-carboxynonadecanoyl))-GLP-1(7-35);
Gly⁸Lys²⁶(N^{ε}-(ω-carboxynonadecanoyl))-GLP-1(7-35);
Gly⁸Lys³⁴(N^{ε}-(ω-carboxynonadecanoyl))-GLP-1(7-35);
Gly⁸Lys^{26,34}-bis(N^{ε}-(ω-carboxynonadecanoyl))-GLP-1(7-35);
Arg²⁶Lys³⁴(N^{ε}-(ω-carboxynonadecanoyl))-GLP-1(7-37);
Gly⁸Arg²⁶Lys³⁴(N^{ε}-(ω-carboxynonadecanoyl))-GLP-1(7-37);
Lys²⁶(N^{ε}-(ω-carboxynonadecanoyl))Arg³⁴-GLP-1(7-37);
Gly⁸Lys²⁶(N^{ε}-(ω-carboxynonadecanoyl))Arg³⁴-GLP-1(7-37);
Arg^{26,34}Lys³⁶(N^{ε}-(ω-carboxynonadecanoyl))-GLP-1(7-37);
Gly⁸Arg^{26,34}Lys³⁶(N^{ε}-(ω-carboxynonadecanoyl))-GLP-1(7-37);
Arg²⁶Lys³⁴(N^{ε}-(ω-carboxynonadecanoyl))-GLP-1(7-38);
Gly⁸Arg²⁶Lys³⁴(N^{ε}-(ω-carboxynonadecanoyl))-GLP-1(7-38);
Lys²⁶(N^{ε}-(ω-carboxynonadecanoyl))Arg³⁴-GLP-1(7-38);
Gly⁸Lys²⁶(N^{ε}-(ω-carboxynonadecanoyl))Arg³⁴-GLP-1(7-38);
Arg^{26,34}Lys³⁶(N^{ε}-(ω-carboxynonadecanoyl))-GLP-1(7-38);
Arg^{26,34}Lys³⁸(N^{ε}-(ω-carboxynonadecanoyl))-GLP-1(7-38);
Gly⁸Arg^{26,34}Lys³⁶(N^{ε}-(ω-carboxynonadecanoyl))-GLP-1(7-38);
Arg²⁶Lys³⁴(N^{ε}-(ω-carboxynonadecanoyl))-GLP-1(7-39);
Gly⁸Arg²⁶Lys³⁴(N^{ε}-(ω-carboxynonadecanoyl))-GLP-1(7-39);
Lys²⁶(N^{ε}-(ω-carboxynonadecanoyl))Arg³⁴-GLP-1(7-39);
Gly⁸Lys²⁶(N^{ε}-(ω-carboxynonadecanoyl))Arg³⁴-GLP-1(7-39);
Arg^{26,34}Lys³⁶(N^{ε}-(ω-carboxynonadecanoyl))-GLP-1(7-39);
Gly⁸Arg^{26,34}Lys³⁶(N^{ε}-(ω-carboxynonadecanoyl))-GLP-1(7-39);
Arg²⁶Lys³⁴(N^{ε}-(ω-carboxynonadecanoyl))-GLP-1(7-40);
Gly⁸Arg²⁶Lys³⁴(N^{ε}-(ω-carboxynonadecanoyl))-GLP-1(7-40);
Lys²⁶(N^{ε}-(ω-carboxynonadecanoyl))Arg³⁴-GLP-1(7-40);
Gly⁸Lys²⁶(N^{ε}-(ω-carboxynonadecanoyl))Arg³⁴-GLP-1(7-40);
Arg^{26,34}Lys³⁶(N^{ε}-(ω-carboxynonadecanoyl))-GLP-1(7-40);
Gly⁸Arg^{26,34}Lys³⁶(N^{ε}-(ω-carboxynonadecanoyl))-GLP-1(7-40);
Lys²⁶(N^{ε}-(7-deoxycholoyl))-GLP-1(7-37);
Lys³⁴(N^{ε}-(7-deoxycholoyl))-GLP-1(7-37);
Lys^{26,34}-bis(N^{ε}-(7-deoxycholoyl))-GLP-1(7-37);
Gly⁸Lys²⁶(N^{ε}-(7-deoxycholoyl))-GLP-1(7-37);
Gly⁸Lys³⁴(N^{ε}-(7-deoxycholoyl))-GLP-1(7-37);
Gly⁸Lys^{26,34}-bis(N^{ε}-(7-deoxycholoyl))-GLP-1(7-37);
Arg²⁶Lys³⁴(N^{ε}-(7-deoxycholoyl))-GLP-1(7-37);
Lys²⁶(N^{ε}-(7-deoxycholoyl))-GLP-1(7-38);
Lys³⁴(N^{ε}-(7-deoxycholoyl))-GLP-1(7-38);
Lys^{26,34}-bis(N^{ε}-(7-deoxycholoyl))-GLP-1(7-38);
Gly⁸Lys²⁶(N^{ε}-(7-deoxycholoyl))-GLP-1(7-38);
Gly⁸Lys³⁴(N^{ε}-(7-deoxycholoyl))-GLP-1(7-38);
Gly⁸Lys^{26,34}-bis(N^{ε}-(7-deoxycholoyl))-GLP-1(7-38);
Arg²⁶Lys³⁴(N^{ε}-(7-deoxycholoyl))-GLP-1(7-38);
Lys²⁶(N^{ε}-(7-deoxycholoyl))-GLP-1(7-39);
Lys³⁴(N^{ε}-(7-deoxycholoyl))-GLP-1(7-39);
Lys^{26,34}-bis(N^{ε}-(7-deoxycholoyl))-GLP-1(7-39);
Gly⁸Lys²⁶(N^{ε}-(7-deoxycholoyl))-GLP-1(7-39);
Gly⁸Lys³⁴(N^{ε}-(7-deoxycholoyl))-GLP-1(7-39);
Gly⁸Lys^{26,34}-bis(N^{ε}-(7-deoxycholoyl))-GLP-1(7-39);
Arg²⁶Lys³⁴(N^{ε}-(7-deoxycholoyl))-GLP-1(7-39);
Lys²⁶(N^{ε}-(7-deoxycholoyl))-GLP-1(7-40);
Lys³⁴(N^{ε}-(7-deoxycholoyl))-GLP-1(7-40);
Lys^{26,34}-bis(N^{ε}-(7-deoxycholoyl))-GLP-1(7-40);
Gly⁸Lys²⁶(N^{ε}-(7-deoxycholoyl))-GLP-1(7-40);
Gly⁸Lys³⁴(N^{ε}-(7-deoxycholoyl))-GLP-1(7-40);
Gly⁸Lys^{26,34}-bis(N^{ε}-(7-deoxycholoyl))-GLP-1(7-40);
Arg²⁶Lys³⁴(N^{ε}-(7-deoxycholoyl))-GLP-1(7-40);
Lys²⁶(N^{ε}-(7-deoxycholoyl))-GLP-1(7-36);
Lys³⁴(N^{ε}-(7-deoxycholoyl))-GLP-1(7-36);
Lys^{26,34}-bis(N^{ε}-(7-deoxycholoyl))-GLP-1(7-36);
Gly⁸Lys²⁶(N^{ε}-(7-deoxycholoyl))-GLP-1(7-36);
Gly⁸Lys³⁴(N^{ε}-(7-deoxycholoyl))-GLP-1(7-36);
Gly⁸Lys^{26,34}-bis(N^{ε}-(7-deoxycholoyl))-GLP-1(7-36);
Arg²⁶Lys³⁴(N^{ε}-(7-deoxycholoyl))-GLP-1(7-36);
Lys²⁶(N^{ε}-(7-deoxycholoyl))-GLP-1(7-35);
Lys³⁴(N^{ε}-(7-deoxycholoyl))-GLP-1(7-35);
Lys^{26,34}-bis(N^{ε}-(7-deoxycholoyl))-GLP-1(7-35);
Gly⁸Lys²⁶(N^{ε}-(7-deoxycholoyl))-GLP-1(7-35);
Gly⁸Lys³⁴(N^{ε}-(7-deoxycholoyl))-GLP-1(7-35);
Gly⁸Lys^{26,34}-bis(N^{ε}-(7-deoxycholoyl))-GLP-1(7-35);
Arg²⁶Lys³⁴(N^{ε}-(7-deoxycholoyl))-GLP-1(7-35);
Lys²⁶(N^{ε}-(7-deoxycholoyl))-GLP-1(7-36)amide;
Lys³⁴(N^{ε}-(7-deoxycholoyl))-GLP-1(7-36)amide;
Lys^{26,34}-bis(N^{ε}-(7-deoxycholoyl))-GLP-1(7-36)amide;
Gly⁸Lys²⁶(N^{ε}-(7-deoxycholoyl))-GLP-1(7-36)amide;
Gly⁸Lys³⁴(N^{ε}-(7-deoxycholoyl))-GLP-1(7-36)amide;
Gly⁸Lys^{26,34}-bis(N^{ε}-(7-deoxycholoyl))-GLP-1(7-36)amide;
Arg²⁶Lys³⁴(N^{ε}-(7-deoxycholoyl))-GLP-1(7-36)amide;
Gly⁸Arg²⁶Lys³⁴(N^{ε}-(7-deoxycholoyl))-GLP-1(7-37);
Lys²⁶(N^{ε}-(7-deoxycholoyl))Arg³⁴-GLP-1(7-37);
Gly⁸Lys²⁶(N^{ε}-(7-deoxycholoyl))Arg³⁴-GLP-1(7-37);
Arg^{26,34}Lys³⁶(N^{ε}-(7-deoxycholoyl))-GLP-1(7-37);
Gly⁸Arg^{26,34}Lys³⁶(N^{ε}-(7-deoxycholoyl))-GLP-1(7-37);
Lys²⁶(N^{ε}-(choloyl))-GLP-1(7-37);
Lys³⁴(N^{ε}-(choloyl))-GLP-1(7-37);
Lys^{26,34}-bis(N^{ε}-(choloyl))-GLP-1(7-37);
Gly⁸Lys²⁶(N^{ε}-(choloyl))-GLP-1(7-37);
Gly⁸Lys³⁴(N^{ε}-(choloyl))-GLP-1(7-37);
Gly⁸Lys^{26,34}-bis(N^{ε}-(choloyl))-GLP-1(7-37);
Arg²⁶Lys³⁴(N^{ε}-(choloyl))-GLP-1(7-37);
Gly⁸Arg²⁶Lys³⁴(N^{ε}-(7-deoxycholoyl))-GLP-1(7-38);
Lys²⁶(N^{ε}-(7-deoxycholoyl))Arg³⁴-GLP-1(7-38);
Gly⁸Lys²⁶(N^{ε}-(7-deoxycholoyl))Arg³⁴-GLP-1(7-38);
Arg^{26,34}Lys³⁶(N^{ε}-(7-deoxycholoyl))-GLP-1(7-38);
Arg^{26,34}Lys³⁸(N^{ε}-(7-deoxycholoyl))-GLP-1(7-38);
Gly⁸Arg^{26,34}Lys³⁶(N^{ε}-(7-deoxycholoyl))-GLP-1(7-38);
Lys²⁶(N^{ε}-(choloyl))-GLP-1(7-38);
Lys³⁴(N^{ε}-(choloyl))-GLP-1(7-38);
Lys^{26,34}-bis(N^{ε}-(choloyl))-GLP-1(7-38);
Gly⁸Lys²⁶(N^{ε}-(choloyl))-GLP-1(7-38);
Gly⁸Lys³⁴(N^{ε}-(choloyl))-GLP-1(7-38);
Gly⁸Lys^{26,34}-bis(N^{ε}-(choloyl))-GLP-1(7-38);
Arg²⁶Lys³⁴(N^{ε}-(choloyl))-GLP-1(7-38);
Gly⁸Arg²⁶Lys³⁴(N^{ε}-(7-deoxycholoyl))-GLP-1(7-39);
Lys²⁶(N^{ε}-(7-deoxycholoyl))Arg³⁴-GLP-1(7-39);
Gly⁸Lys²⁶(N^{ε}-(7-deoxycholoyl))Arg³⁴-GLP-1(7-39);
Arg^{26,34}Lys³⁶(N^{ε}-(7-deoxycholoyl))-GLP-1(7-39);
Gly⁸Arg^{26,34}Lys³⁶(N^{ε}-(7-deoxycholoyl))-GLP-1(7-39);
Lys²⁶(N^{ε}-(choloyl))-GLP-1(7-39);
Lys³⁴(N^{ε}-(choloyl))-GLP-1(7-39);
Lys^{26,34}-bis(N^{ε}-(choloyl))-GLP-1(7-39);
Gly⁸Lys²⁶(N^{ε}-(choloyl))-GLP-1(7-39);
Gly⁸Lys³⁴(N^{ε}-(choloyl))-GLP-1(7-39);
Gly⁸Lys^{26,34}-bis(N^{ε}-(choloyl))-GLP-1(7-39);
Arg²⁶Lys³⁴(N^{ε}-(choloyl))-GLP-1(7-39);
Gly⁸Arg²⁶Lys³⁴(N^{ε}-(7-deoxycholoyl))-GLP-1(7-40);
Lys²⁶(N^{ε}-(7-deoxycholoyl))Arg³⁴-GLP-1(7-40);
Gly⁸Lys²⁶(N^{ε}-(7-deoxycholoyl))Arg³⁴-GLP-1(7-40);
Arg^{26,34}Lys³⁶(N^{ε}-(7-deoxycholoyl))-GLP-1(7-40);
Gly⁸Arg^{26,34}Lys³⁶(N^{ε}-(7-deoxycholoyl))-GLP-1(7-40);
Lys²⁶(N^{ε}-(choloyl))-GLP-1(7-40);
Lys³⁴(N^{ε}-(choloyl))-GLP-1(7-40);
Lys^{26,34}-bis(N^{ε}-(choloyl))-GLP-1(7-40);
Gly⁸Lys²⁶(N^{ε}-(choloyl))-GLP-1(7-40);
Gly⁸Lys³⁴(N^{ε}-(choloyl))-GLP-1(7-40);
Gly⁸Lys^{26,34}-bis(N^{ε}-(choloyl))-GLP-1(7-40);
Arg²⁶Lys³⁴(N^{ε}-(choloyl))-GLP-1(7-40);
Lys²⁶(N^{ε}-(choloyl))-GLP-1(7-36);
Lys³⁴(N^{ε}-(choloyl))-GLP-1(7-36);
Lys^{26,34}-bis(N^{ε}-(choloyl))-GLP-1(7-36);
Gly⁸Lys²⁶(N^{ε}-(choloyl))-GLP-1(7-36);
Gly⁸Lys³⁴(N^{ε}-(choloyl))-GLP-1(7-36);
Gly⁸Lys^{26,34}-bis(N^{ε}-(choloyl))-GLP-1(7-36);
Arg²⁶Lys³⁴(N^{ε}-(choloyl))-GLP-1(7-36);
Lys²⁶(N^{ε}-(choloyl))-GLP-1(7-35);
Lys³⁴(N^{ε}-(choloyl))-GLP-1(7-35);
Lys^{26,34}-bis(N^{ε}-(choloyl))-GLP-1(7-35);
Gly⁸Lys²⁶(N^{ε}-(choloyl))-GLP-1(7-35);
Gly⁸Lys³⁴(N^{ε}-(choloyl))-GLP-1(7-35);
Gly⁸Lys^{26,34}-bis(N^{ε}-(choloyl))-GLP-1(7-35);
Arg²⁶Lys³⁴(N^{ε}-(choloyl))-GLP-1(7-35);
Lys²⁶(N^{ε}-(choloyl))-GLP-1(7-36)amide;
Lys³⁴(N^{ε}-(choloyl))-GLP-1(7-36)amide;
Lys^{26,34}-bis(N^{ε}-(choloyl))-GLP-1(7-36)amide;
Gly⁸Lys²⁶(N^{ε}-(choloyl))-GLP-1(7-36)amide;
Gly⁸Lys³⁴(N^{ε}-(choloyl))-GLP-1(7-36)amide;
Gly⁸Lys^{26,34}-bis(N^{ε}-(choloyl))-GLP-1(7-36)amide;
Arg²⁶Lys³⁴(N^{ε}-(choloyl))-GLP-1(7-36)amide;
Gly⁸Arg²⁶Lys³⁴(N^{ε}-(choloyl))-GLP-1(7-37);
Lys²⁶(N^{ε}-(choloyl))Arg³⁴-GLP-1(7-37);
Gly⁸Lys²⁶(N^{ε}-(choloyl))Arg³⁴-GLP-1(7-37);
Arg^{26,34}Lys³⁶(N^{ε}-(choloyl))-GLP-1(7-37);
Gly⁸Arg^{26,34}Lys³⁶(N^{ε}-(choloyl))-GLP-1(7-37);
Lys²⁶(N^{ε}-(lithocholoyl))-GLP-1(7-37);
Lys³⁴(N^{ε}-(lithocholoyl))-GLP-1(7-37);
Lys^{26,34}-bis(N^{ε}-(lithocholoyl))-GLP-1(7-37);
Gly⁸Lys²⁶(N^{ε}-(lithocholoyl))-GLP-1(7-37);
Gly⁸Lys³⁴(N^{ε}-(lithocholoyl))-GLP-1(7-37);
Gly⁸Lys^{26,34}-bis(N^{ε}-(lithocholoyl))-GLP-1(7-37);
Arg²⁶Lys³⁴(N^{ε}-(lithocholoyl))-GLP-1(7-37);
Gly⁸Arg²⁶Lys³⁴(N^{ε}-(choloyl))-GLP-1(7-38);
Lys²⁶(N^{ε}-(choloyl))Arg³⁴-GLP-1(7-38);
Gly⁸Lys²⁶(N^{ε}-(choloyl))Arg³⁴-GLP-1(7-38);
Arg^{26,34}Lys³⁶(N^{ε}-(choloyl))-GLP-1(7-38);
Arg^{26,34}Lys³⁸(N^{ε}-(choloyl))-GLP-1(7-38);
Gly⁸Arg^{26,34}Lys³⁶(N^{ε}-(choloyl))-GLP-1(7-38);
Lys²⁶(N^{ε}-(lithocholoyl))-GLP-1(7-38);
Lys³⁴(N^{ε}-(lithocholoyl))-GLP-1(7-38);
Lys^{26,34}-bis(N^{ε}-(lithocholoyl))-GLP-1(7-38);
Gly⁸Lys²⁶(N^{ε}-(lithocholoyl))-GLP-1(7-38);
Gly⁸Lys³⁴(N^{ε}-(lithocholoyl))-GLP-1(7-38);
Gly⁸Lys^{26,34}-bis(N^{ε}-(lithocholoyl))-GLP-1(7-38);
Arg²⁶Lys³⁴(N^{ε}-(lithocholoyl))-GLP-1(7-38);
Gly⁸Arg²⁶Lys³⁴(N^{ε}-(choloyl))-GLP-1(7-39);
Lys²⁶(N^{ε}-(choloyl))Arg³⁴-GLP-1(7-39);
Gly⁸Lys²⁶(N^{ε}-(choloyl))Arg³⁴-GLP-1(7-39);
Arg^{26,34}Lys³⁶(N^{ε}-(choloyl))-GLP-1(7-39);
Gly⁸Arg^{26,34}Lys³⁶(N^{ε}-(choloyl))-GLP-1(7-39);
Lys²⁶(N^{ε}-(lithocholoyl))-GLP-1(7-39);
Lys³⁴(N^{ε}-(lithocholoyl))-GLP-1(7-39);
Lys^{26,34}-bis(N^{ε}-(lithocholoyl))-GLP-1(7-39);
Gly⁸Lys²⁶(N^{ε}-(lithocholoyl))-GLP-1(7-39);
Gly⁸Lys³⁴(N^{ε}-(lithocholoyl))-GLP-1(7-39);
Gly⁸Lys^{26,34}-bis(N^{ε}-(lithocholoyl))-GLP-1(7-39);
Arg²⁶Lys³⁴(N^{ε}-(lithocholoyl))-GLP-1(7-39);
Gly⁸Arg²⁶Lys³⁴(N^{ε}-(choloyl))-GLP-1(7-40);
Lys²⁶(N^{ε}-(choloyl))Arg³⁴-GLP-1(7-40);
Gly⁸Lys²⁶(N^{ε}-(choloyl))Arg³⁴-GLP-1(7-40);
Arg^{26,34}Lys³⁶(N^{ε}-(choloyl))-GLP-1(7-40);
Gly⁸Arg^{26,34}Lys³⁶(N^{ε}-(choloyl))-GLP-1(7-40);
Lys²⁶(N^{ε}-(lithocholoyl))-GLP-1(7-40);
Lys³⁴(N^{ε}-(lithocholoyl))-GLP-1(7-40);
Lys^{26,34}-bis(N^{ε}-(lithocholoyl))-GLP-1(7-40);
Gly⁸Lys²⁶(N^{ε}-(lithocholoyl))-GLP-1(7-40);
Gly⁸Lys³⁴(N^{ε}-(lithocholoyl))-GLP-1(7-40);
Gly⁸Lys^{26,34}-bis(N^{ε}-(lithocholoyl))-GLP-1(7-40);
Arg²⁶Lys³⁴(N^{ε}-(lithocholoyl))-GLP-1(7-37);
Lys²⁶(N^{ε}-(lithocholoyl))-GLP-1(7-36);
Lys³⁴(N^{ε}-(lithocholoyl))-GLP-1(7-36);
Lys^{26,34}-bis(N^{ε}-(lithocholoyl))-GLP-1(7-36);
Gly⁸Lys²⁶(N^{ε}-(lithocholoyl))-GLP-1(7-36);
Gly⁸Lys³⁴(N^{ε}-(lithocholoyl))-GLP-1(7-36);
Gly⁸Lys^{26,34}-bis(N^{ε}-(lithocholoyl))-GLP-1(7-36);
Arg²⁶Lys³⁴(N^{ε}-(lithocholoyl))-GLP-1(7-36);
Lys²⁶(N^{ε}-(lithocholoyl))-GLP-1(7-35);
Lys³⁴(N^{ε}-(lithocholoyl))-GLP-1(7-35);
Lys^{26,34}-bis(N^{ε}-(lithocholoyl))-GLP-1(7-35);
Gly⁸Lys²⁶(N^{ε}-(lithocholoyl))-GLP-1(7-35);
Gly⁸Lys³⁴(N^{ε}-(lithocholoyl))-GLP-1(7-35);
Gly⁸Lys^{26,34}-bis(N^{ε}-(lithocholoyl))-GLP-1(7-35);
Arg²⁶Lys³⁴(N^{ε}-(lithocholoyl))-GLP-1(7-35);
Lys²⁶(N^{ε}-(lithocholoyl))-GLP-1(7-36)amide;
Lys³⁴(N^{ε}-(lithocholoyl))-GLP-1(7-36)amide;
Lys^{26,34}-bis(N^{ε}-(lithocholoyl))-GLP-1(7-36)amide;
Gly⁸Lys²⁶(N^{ε}-(lithocholoyl))-GLP-1(7-36)amide;
Gly⁸Lys³⁴(N^{ε}-(lithocholoyl))-GLP-1(7-36)amide;
Gly⁸Lys^{26,34}-bis(N^{ε}-(lithocholoyl))-GLP-1(7-36)amide;
Arg²⁶Lys³⁴(N^{ε}-(lithocholoyl))-GLP-1(7-36)amide;
Gly⁸Arg²⁶Lys³⁴(N^{ε}-(lithocholoyl))-GLP-1(7-37);
Lys²⁶(N^{ε}-(lithocholoyl))Arg³⁴-GLP-1(7-37);
Gly⁸Lys²⁶(N^{ε}-(lithocholoyl))Arg³⁴-GLP-1(7-37);
Arg^{26,34}Lys³⁶(N^{ε}-(lithocholoyl))-GLP-1(7-37);
Arg^{26,34}Lys³⁸(N^{ε}-(lithocholoyl))-GLP-1(7-37);
Gly⁸Arg^{26,34}Lys³⁶(N^{ε}-(lithocholoyl))-GLP-1(7-37);
Gly⁸Arg²⁶Lys³⁴(N^{ε}-(lithocholoyl))-GLP-1(7-38);
Lys²⁶(N^{ε}-(lithocholoyl))Arg³⁴-GLP-1(7-38);
Gly⁸Lys²⁶(N^{ε}-(lithocholoyl))Arg³⁴-GLP-1(7-38);
Arg^{26,34}Lys³⁶(N^{ε}-(lithocholoyl))-GLP-1(7-38);
Arg^{26,34}Lys³⁸(N^{ε}-(lithocholoyl))-GLP-1(7-38); Gly⁸Arg^{26,34}Lys³⁶(N^{ε}-(lithocholoyl))-GLP-1(7-38);
Gly⁸Arg²⁶Lys³⁴(N^{ε}-(lithocholoyl))-GLP-1(7-39);
Lys²⁶(N^{ε}-(lithocholoyl))Arg³⁴-GLP-1(7-39);
Gly⁸Lys²⁶(N^{ε}-(lithocholoyl))Arg³⁴-GLP-1(7-39);
Arg^{26,34}Lys³⁶(N^{ε}-(lithocholoyl))-GLP-1(7-39);
Gly⁸Arg^{26,34}Lys³⁶(N^{ε}-(lithocholoyl))-GLP-1(7-39);
Gly⁸Arg²⁶Lys³⁴(N^{ε}-(lithocholoyl))-GLP-1(7-40);
Lys²⁶(N^{ε}-(lithocholoyl))Arg³⁴-GLP-1(7-40);
Gly⁸Lys²⁶(N^{ε}-(lithocholoyl))Arg³⁴-GLP-1(7-40);
Arg^{26,34}Lys³⁶(N^{ε}-(lithocholoyl))-GLP-1(7-40) and
Gly⁸Arg^{26,34}Lys³⁶(N^{ε}-(lithocholoyl))-GLP-1(7-40). Each one of these specific GLP-1 derivatives constitutes an alternative embodiment of the invention.

A more preferred GLP-1 derivative is Arg³⁴, Lys²⁶(N^{ε}-(γ-Glu(N^{α}-hexadecanoyl)))-GLP-1 (7-37). Another more preferred GLP-1 derivative is Arg²⁶, Lys³⁴(N-ε-(γ-Glu(N-α-hexadecanoyl)))-GLP-1(7-37).

GLP-1 analogues and derivatives which can be used according to the present invention includes those referred to in WO 99/43705 (Novo Nordisk A/S), WO 99/43706 (Novo Nordisk A/S), WO 99/43707 (Novo Nordisk A/S), WO 98/08871 (Novo Nordisk A/S), WO 99/43708 (Novo Nordisk A/S), WO 99/43341 (Novo Nordisk A/S), WO 87/06941 (The General Hospital Corporation), WO 90/11296 (The General Hospital Corporation), WO 91/11457 (Buckley et al.), WO 98/43658 (Eli Lilly & Co.), EP 0708179-A2 (Eli Lilly & Co.), EP 0699686-A2 (Eli Lilly & Co.) which are included herein by reference.

However, protracted acting GLP-1 derivatives, in particular those described in WO 98/08871 are more preferred. The most preferred GLP-1 derivatives are those in which the parent peptide has the formula GLP-1 (7-C), wherein C is 36, 37, 38, 39, 40, 41, 42, 43, 44 and 45, wherein optionally a total of up to fifteen, preferably up to ten amino acid residues have been exchanged with any α-amino acid residue which can be coded for by the genetic code, said parent peptide comprising one or two lipophilic substituents having 4 to 40 carbon atoms, preferably from 8 to 25 carbon atoms, optionally via a spacer (such as γ-Glu or β-Ala). The substituents are preferably selected from acyl groups of straight-chained or branched fatty acids.

GLP-1 analogues and derivatives that include an N-terminal imidazole group and optionally an unbranched C₆-C₁₀ acyl group attached to the lysine residue in position 34 are also embodiments of the invention.

In a still further embodiment of the invention the GLP-1 agonist is selected from exendin as well as analogs, derivatives, and fragments thereof, e.g. exendin-3 and -4.

Examples of exendin as well as analogs, derivatives, and fragments thereof to be included within the present invention are those disclosed in WO 9746584 and US 5424286. US 5424286 describes a method for stimulating insulin release with exendin polypeptide(s). The exendin polypeptides disclosed include HGEGTFTSDLSKQMEEEAVRLFIEWLKNGGX; wherein X = P or Y, and HX1 X2GTFITSDLSKQMEEEAVRLFIEWLKNGGPSSGAPPPS; wherein X1X2 = SD (exendin-3) or GE (exendin-4)). The exendin-3 and -4 and fragments are useful in treatment of diabetes mellitus (types I or II) and prevention of hyperglycaemia. They normalise hyperglycaemia through glucose-dependent, insulin-independent and insulin-dependent mechanisms. Exendin-4 is specific for exendin receptors, i.e. it does not interact with vasoactive intestinal peptide receptors. WO 9746584 describes truncated versions of exendin peptide(s) for treating diabetes. The disclosed peptides increase secretion and biosynthesis of insulin, but reduce those of glucagon. The truncated peptides can be made more economically than full length versions.

In a still further embodiment of the invention the GLP-1 agonist is a non-peptide. In a further embodiment the GLP-1 agonist binds to a GLP-1 receptor with an affinity constant, K_{D}, below 1 µM, preferably below 100 nM.

In a still further embodiment the GLP-1 agonist is a non-peptide, which binds to a GLP-1 receptor with an affinity constant, K_{D}, below 1 µM, preferably below 100 nM.

Any possible combination of two or more of the embodiments described herein, is comprised within the scope of the present invention. The term "GLP-1" is intended to mean GLP-1 (7-37) or GLP-1 (7-36) amide.

The term "treatment" is defined as the management and care of a patient for the purpose of combating the disease, condition, or disorder and includes the administration of a GLP-1 agonist to prevent the onset of the symptoms or complications, or alleviating the symptoms or complications, or eliminating the disease, condition, or disorder.

In the present context "a GLP-1 agonist" is intended to indicate a molecule, preferably GLP-1 or an analogue or a derivative thereof, or exendin or an analogue or a derivative thereof, or a non-peptide, which binds to a GLP-1 receptor with an affinity constant, K_{D}, below 1 µM, preferably below 100 nM. Methods for identifying GLP-1 agonists are described in WO 93/19175 (Novo Nordisk A/S).

In the present context "a GLP-1 agonist" is also intended to comprise active metabolites and prodrugs thereof, such as active metabolites and prodrugs of GLP-1 or an analogue or a derivative thereof, or exendin or an analogue or a derivative thereof, or a non-peptide. A "metabolite" is an active derivative of a GLP-1 agonist produced when the GLP-1 agonist is metabolized. A "prodrug" is a compound which is either metabolized to a GLP-1 agonist or is metabolized to the same metabolite(s) as a GLP-1 agonist.

In the present text, the designation "an analogue" is used to designate a peptide wherein one or more amino acid residues of the parent peptide have been substituted by another amino acid residue and/or wherein one or more amino acid residues of the parent peptide have been deleted and/or wherein one or more amino acid residues have been added to the parent peptide. Such addition can take place either in the peptide, at the N-terminal end or at the C-terminal end of the parent peptide, or any combination thereof.

The term "derivative" is used in the present text to designate a peptide in which one or more of the amino acid residues of the parent peptide have been chemically modified, e.g. by alkylation, acylation, ester formation or amide formation.

The term "a GLP-1 derivative" is used in the present text to designate a derivative of GLP-1 or an analogue thereof. In the present text, the parent peptide from which such a derivative is formally derived is in some places referred to as the "GLP-1 moiety" of the derivative.

In the present specification "premature occlusive arterial disease" is intended to comprise those disease-states, which are characterized by the closure of an artery or the progress of closing an artery, and which a) develop earlier in life than normal and b) are associated with high levels of Lp(a) in the plasma; and includes, i.a., stroke, coronary artery disease, atherosclerosis, arteriosclerosis, myocardial infarction, restenosis, peripheral artery disease and bypass graft stenosis.

For a description of suitable dosage forms, dosage ranges, pharmaceutical formulations etc. reference is made to eg. WO 91/11457, WO 98/08871, WO 9746584, or US 5424286.

The route of administration may be any route, which effectively transports the active compound to the appropriate or desired site of action, such as oral, nasal, pulmonary, transdermal or parenteral.

Pharmaceutical compositions (or medicaments) containing a GLP-1 agonist, may be administered parenterally to patients in need of such a treatment. Parenteral administration may be performed by subcutaneous, intramuscular or intravenous injection by means of a syringe, optionally a pen-like syringe. Alternatively, parenteral administration can be performed by means of an infusion pump. A further option is a composition which may be a powder or a liquid for the administration of the GLP-1 agonist in the form of a nasal or pulmonal spray. As a still further option, the GLP-1 agonist can also be administered transdermally, e.g. from a patch, optionally a iontophoretic patch, or transmucosally, e.g. bucally. As a still further option, the GLP-1 agonist (in particular GLP-1 or an analogue thereof) can also be administered by gene therapy, such as by implanting a cell line transformed with a vector such that it secretes the GLP-1 agonist. The implanted cells may be encapsulated in semi permeable membranes, e.g. macro- or microencapsulated. The above mentioned possible ways to administer a GLP-1 agonist are not considered as limiting the scope of the invention.

Pharmaceutical compositions containing a GLP-1 agonist may be prepared by conventional techniques, *e*.*g*. as described in Remington's *Pharmaceutical Sciences,* 1985 or in Remington: *The Science and Practice of Pharmacy,* 19^{th} edition, 1995.

Thus, the injectable compositions of the GLP-1 agonist can be prepared using the conventional techniques of the pharmaceutical industry which involves dissolving and mixing the ingredients as appropriate to give the desired end product.

According to one procedure, the GLP-1 agonist is dissolved in an amount of water which is somewhat less than the final volume of the composition to be prepared. An isotonic agent, a preservative and a buffer is added as required and the pH value of the solution is adjusted - if necessary - using an acid, e.g. hydrochloric acid, or a base, e.g. aqueous sodium hydroxide as needed. Finally, the volume of the solution is adjusted with water to give the desired concentration of the ingredients.

Examples of isotonic agents are sodium chloride, mannitol and glycerol. Examples of preservatives are phenol, m-cresol, methyl p-hydroxybenzoate and benzyl alcohol.

Examples of suitable buffers are sodium acetate and sodium phosphate. Further to the above-mentioned components, solutions containing a GLP-1 agonist may also contain a surfactant in order to improve the solubility and/or the stability of the GLP-1 agonist.

A composition for nasal administration of certain peptides may, for example, be prepared as described in European Patent No. 272097 (to Novo Nordisk A/S) or in WO 93/18785.

According to one embodiment of the present invention, the GLP-1 agonist is provided in the form of a composition suitable for administration by injection. Such a composition can either be an injectable solution ready for use or it can be an amount of a solid composition, e.g. a lyophilised product, which has to be dissolved in a solvent before it can be injected. The injectable solution preferably contains not less than about 2 mg/ml, preferably not less than about 5 mg/ml, more preferred not less than about 10 mg/ml of the GLP-1 agonist and, preferably, not more than about 100 mg/ml of the GLP-1 agonist.

The GLP-1 agonist can be used in the treatment of various diseases. The particular GLP-1 agonist to be used and the optimal dose level for any patient (effective amount) will depend on the disease to be treated and on a variety of factors including the efficacy of the specific peptide derivative employed, the age, body weight, physical activity, and diet of the patient, on a possible combination with other drugs, and on the severity of the case. It is recommended that the dosage of the GLP-1 agonist be determined for each individual patient by those skilled in the art.

### Experimental

### Example 1

Acute and subchronic anorectic effects of Arg³⁴, Lys²⁶(N^{ε}-(γ-Glu(N^{α}-hexadecanoyl)))-GLP-1 (7-37) (hereinafter GLP1) were studied in both normal Wistar rats and a model of hypothalamic obesity (Wistar rats subjected to neonatal monosodium glutamate treatment (MSG)). MSG lesioned rats were randomly assigned to groups receiving GLP1 or vehicle. 100 µg/kg GLP1 was administered by subcutaneous injection twice daily. GLP1 was dissolved in sterile phosphate buffered saline (50 mM, pH 7.4) to a final concentration of either 0.1 or 1 mg/ml. Solutions were always made fresh approximately 1 hour prior to use and stored at 4°C in sterile tubes. An inhibition of food intake was observed, accompanied by reduced body weight. Initial decreases in water intake and increases in diuresis were normalised within few days of treatment, and plasma parameters of renal function remained normal throughout the experiment. Lowered plasma levels of triglycerides were observed. In normal rats from 1.58±0.1 to 1.21±0.3 mM and in MSG rats from 2.27±0.3 to 1.49±0.1 mM.

### Example 2

The male Zucker Diabetic Fatty fa/fa (ZDF) rat is a model of Type 2 diabetes. The rats are insulin resistant but normoglycemic from birth and they develop diabetes from about week 7 to week 10 of age. During the transitional period, the animals go through a state of impaired glucose tolerance. Although the animals are hyperinsulinemic before diabetes onset and during the early stages of diabetes, they later lose glucose-stimulated insulin secretion and finally become almost completely insulinopenic.

We have studied the effects of GLP1 therapy during a period of time when the animals would normally progress from having impaired glucose tolerance to having overt Type 2 diabetes. Three groups of male ZDF rats (Genetic Models Inc, Indianapolis, Indiana, USA) were studied and dosed subcutaneously bi-daily with either vehicle (group A), 30 (group B) or 150 µg/kg (group C) of GLP1, n=6 per group. Animals were between 7 and 8 weeks old when dosing was initiated, and fed glucose levels were not different between the groups before dosing began. However, they were elevated compared to a group of non-diabetic Sprague-Dawley rats who had fed glucose levels significantly below the ZDF animals (6.4±0.6 vs 5.8±0.8, mean±SD, p<0.02). This demonstrates the relative impaired glucose tolerant state of the ZDF animals when the study began. After 10 days of dosing, group C had blood glucose levels during a normal 24-hour feeding schedule that were unchanged compared to the initial measurements and they were significantly lower than the vehicle- and low-dose-treated animals who were hyperglycemic. After 36 days of dosing, an oral glucose tolerance test was performed in the animals after an 11-hour fast. One g/kg of glucose was administered by oral gavage and subsequent measurements of blood glucose and plasma insulin were made. Also in this test, the glycemic level was significantly lower in group C compared to groups A and B. These results demonstrate that treatment with GLP1 can prevent or delay the progression of impaired glucose tolerance to Type 2 diabetes.

In addition to the effects on blood glucose and insulin, we also studied the effects of long-term treatment on lipid parameters. Thus, at the termination of the study, non-fasted plasma measurements of total cholesterol, free fatty acids were performed. The results, summarized in Table 1, demonstrated a significant reduction in free fatty acids and total cholesterol in group C compared to group A. This demonstrates that treatment with GLP1 has lipid-lowering properties.

**Table 1 Lipid parameters. Data are expressed as mean±SEM**

| | Free fatty acids (mM) | Total cholesterol (mM) |
|---|---|---|
| Group A | 0.34±0.01 | 2.8±0.1 |
| Group B | 0.29±0.04 | 2.9±0.1 |
| Group C | 0.20±0.02 | 2.2±0.1 |

## Claims

1. The a use of a GLP-1 agonist for the manufacture of a medicament for treating dyslipidemia in a patient having type 2 diabetes, in a regimen which additionally comprises treatment with a biguanide.

2. The use according to claim 1, wherein biguanide is metformin.

3. The use of a GLP-1 agonist for the manufacture of a medicament for treating dyslipidemia in a patient having type 2 diabetes, in a regimen which additionally comprises treatment with repaglinide.

4. The use of a GLP-1 agonist for the manufacture of a medicament for treating dyslipidemia in a patient having type 2 diabetes, in a regimen which additionally comprises treatment with an α-glucosidase inhibitor such as any one of miglitol or acarbose.

5. The use of a GLP-1 agonist, for the manufacture of a medicament for treating dyslipidemia in a patient having type 2 diabetes, in a regimen which additionally comprises treatment with a thiazolidinedione.

6. The use according to claim 5, wherein the thiazolidinedione are any one of troglitazone, ciglitazone, pioglitazone or rosiglitazone.

7. The use of a GLP-1 agonist for the manufacture of a medicament for the treatment of dyslipidemia, which are administered in combination with an antihyperlipidemic agent or antilipidemic agent.

8. The use according to claim 7, wherein the antihyperlipidemic agent or antilipidemic agent is selected from cholestyramine, colestipol, clofibrate, gemfibrozil, lovastatin, pravastatin, simvastatin, probucol or dextrothyroxine.

9. The use of a GLP-1 agonist for the manufacture of a medicament for treating dyslipidemia in a patient having type 2 diabetes, in a regimen which additionally comprises treatment with insulin.

10. The use of a GLP-1 agonist for the manufacture of a medicament for treating dyslipidemia in a patient having type 2 diabetes, in a regimen which additionally comprises treatment with a sulphonylurea, such as any one of tolbutamide, glibenclamide, glipizide or glicazide.

11. The use of a GLP-1 agonist for the manufacture of a medicament, which may be administered in combination with more than one of the above-mentioned compounds in combination.

12. The use according to claim 11, in which the GLP-1 agonist is administered with a sulphonylurea and metformin

13. The use according to claim 11, in which the GLP-1 agonist is administered with a sulphonylurea and acarbose

14. The use according to claim 11, in which the GLP-1 agonist is administered with repaglinide and metformin

15. The use according to claim 11, in which the GLP-1 agonist is administered with insulin and a sulphonylurea,

16. The use according to claim 11, in which the GLP-1 agonist is administered with insulin and metformin.

17. The use according to claim 11, in which the GLP-1 agonist is administered with insulin and troglitazone.

18. The use according to claim 11, in which the GLP-1 agonist is administered with a insulin and lovastatin.

19. The use according to any one of claims 1-18 wherein the GLP-1 agonist binds to a GLP-1 receptor with an affinity constant, K_{D}, below 1 µM.

20. The use according to any one of claims 1-18 wherein the GLP-1 agonist is selected from Arg²⁶, Lys³⁴(N-ε-(γ-Glu(N-α-hexadecanoyl)))-GLP-1(7-37), Arg³⁴, Lys²⁶(N-ε-(γ-Glu(N-α-hexadecanoyl)))-GLP-1 (7-37), exendin-3, exendin-4, Val⁸-GLP-1(7-37), Thr⁸- GLP-1 (7-37), Met⁸- GLP-1 (7-37), Gly⁸-GLP-1(7-37).

21. The use according to any one of the claims 1-18 wherein the GLP-1 agonist is Arg³⁴, Lys²⁶(N^{ε}-(γ-Glu(N^{α}-hexadecanoyl)))-GLP-1(7-37).
